# EUROPEAN PATENT APPLICATION

(11) **EP 4 487 875 A1**
(43) Date of publication of application: **08.01.2025**
(21) Application number: 23760129.9
(22) Date of filing: 24.02.2023
(51) Int. Cl.: A61K 48/00, A61K 31/7105, A61K 35/76, A61K 38/46, A61P 27/02, C12N 15/09, C12N 15/55, C12N 15/63, C12N 15/864

(54) **MEDICINE FOR DISEASE CAUSED BY FRAME-SHIFT MUTATION**

(30) Priority: 28.02.2022 JP 2022030349
(71) Applicant: National University Corporation Tokai National Higher Education and Research System, Nagoya-shi, Aichi 464-8601 (JP); JCR Pharmaceuticals Co., Ltd., Ashiya-shi, Hyogo 659-0021 (JP)
(72) Inventor: NISHIGUCHI, Koji, Nagoya-shi, Aichi 464-8601 (JP); FUJITA, Kosuke, Nagoya-shi, Aichi 464-8601 (JP); ASHIDA, Yuhei, Kobe-shi, Hyogo 651-2241 (JP)
(74) Representative: Vossius & Partner Patentanwälte Rechtsanwälte mbB
(86) International application number: PCT/JP2023/006843
(87) International publication number: WO 2023/163131

(57) **Abstract**

The present disclosure provides a medicine for treating or preventing a hereditary disease, etc. The present disclosure provides a medicine for altering, preventing, or treating a condition, disease, disorder or symptom, which is caused by a frame-shift mutation due to the insertion or deletion of one or more bases, by cleaving at least one a target nucleic acid sequence in a nucleic acid within a cell, said medicine comprising a vector. This vector contains a construct that contains a promoter specific to the cell, a sequence encoding a Cas nuclease, a promoter enabling the expression of a gRNA in the cell after the introduction of the vector, and a sequence encoding a gRNA that binds to a target nucleic acid sequence having the insertion or deletion. This construct is configured so as to form a cleavage site, by the Cas nuclease, in proximity to the target nucleic acid sequence having the insertion or deletion.

## Description

### [Technical Field]

The present disclosure relates to a medicament, a construct, and a method for altering, preventing, or treating a condition, disease, disorder, or symptom caused by a frameshift mutation due to insertion or deletion of one or more bases by cleaving at least one site of a target nucleic acid sequence in a nucleic acid within a cell.

### [Background Art]

Hereditary diseases result from aberrant expression of a gene due to some abnormality in the gene such as reduced or lost expression of the gene or increased expression of the gene. Among such hereditary diseases, a nonsense mutation type hereditary disease is a disease caused by a point mutation on a gene that results in formation of a premature termination codon and thus inhibition of protein expression. If a termination codon appears upstream (on a 5' end side of) the original termination codon on an mRNA, a protein translated therefrom is shortened, that is, no functional protein is obtained, resulting in appearance of the hereditary disease.

A frameshift mutation may also cause a termination codon to appear in a location other than its original location, and deletion or insertion of bases in a number other than a multiple of 3 in a gene may shift a reading frame during translation, resulting in a hereditary disease.

### [Summary of Invention]

### [Solution to Problem]

Therefore, the present disclosure provides the following items.
(Item 1)
   A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament including:
   a Cas nuclease and/or a nucleic acid including a nucleic acid sequence encoding a Cas nuclease; and
   a nucleic acid including a nucleic acid sequence of a gRNA binding to the target nucleic acid sequence having the insertion or deletion,
   the Cas nuclease producing a cleavage site in the proximity of the target nucleic acid sequence having the insertion or deletion.
(Item 2)
   The medicament according to the above item, in which the medicament includes a vector, the vector includes a construct including a promoter specific to the cell, a sequence encoding the Cas nuclease, a promoter enabling expression of a gRNA in the cell after introduction of the vector, and a sequence encoding a gRNA binding to the target nucleic acid sequence having the insertion or deletion, and
   the construct is configured for the Cas nuclease to produce a cleavage site in the proximity of the target nucleic acid sequence having the insertion or deletion.
(Item 3)
   The medicament according to any one of the above items, in which the cleavage site is flanked by a site of the insertion or deletion.
(Item 4)
   The medicament according to any one of the above items, in which the condition, disease, disorder, or symptom caused by a frameshift mutation includes retinitis pigmentosa caused by an EYS S1653K frameshift mutation.
(Item 5)
   The medicament according to any one of the above items, in which the vector includes an adeno-associated virus (AAV) vector.
(Item 6)
   The medicament according to any one of the above items, in which the gRNA binding to the target nucleic acid sequence having the insertion or deletion satisfies a predetermined score in a gRNA design tool.
(Item 6a)
   The medicament according to any one of the above items, in which the gRNA binding to the target nucleic acid sequence having the insertion or deletion exhibits a value of a score indicating editing efficiency of about 50% or more in the gRNA design tool.
(Item 6b)
   The medicament according to any one of the above items, in which the gRNA binding to the target nucleic acid sequence having the insertion or deletion exhibits a value of a score indicating specificity for the target nucleic acid sequence of about 90% or more in the gRNA design tool.
(Item 6c)
   The medicament according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item A1)
   A construct for altering, preventing, or treating a condition, disease, disorder, or symptom caused by a frameshift mutation due to insertion or deletion of one or more bases by cleaving at least one site of a target nucleic acid sequence in a nucleic acid within a cell, the construct being included in a vector, the construct including a promoter specific to the cell, a sequence encoding a Cas nuclease, a promoter enabling expression of a gRNA in the cell after introduction of the vector, and a sequence encoding a gRNA binding to the target nucleic acid sequence having the insertion or deletion, and
   the construct being configured for the Cas nuclease to produce a cleavage site in the proximity of the target nucleic acid sequence having the insertion or deletion.
(Item A2)
   The construct according to any one of the above items, in which the cleavage site is flanked by a site of the insertion or deletion.
(Item A3)
   The construct according to any one of the above items, in which the condition, disease, disorder, or symptom caused by a frameshift mutation includes retinitis pigmentosa caused by an EYS S1653K frameshift mutation.
(Item A4)
   The construct according to any one of the above items, in which the vector includes an adeno-associated virus (AAV) vector.
(Item A5)
   The construct according to any one of the above items, in which the gRNA binding to the target nucleic acid sequence having the insertion or deletion satisfies a predetermined score in a gRNA design tool.
(Item A5a)
   The construct according to any one of the above items, in which the gRNA binding to the target nucleic acid sequence having the insertion or deletion exhibits a value of a score indicating editing efficiency of about 50% or more in the gRNA design tool.
(Item A5b)
   The construct according to any one of the above items, in which the gRNA binding to the target nucleic acid sequence having the insertion or deletion exhibits a value of a score indicating specificity for the target nucleic acid sequence of about 90% or more in the gRNA design tool.
(Item A5c)
   The construct according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item B1)
   A construct for altering, preventing, or treating a condition, disease, disorder, or symptom caused by a frameshift mutation due to insertion or deletion of one or more bases by cleaving at least one site of a target nucleic acid sequence in a nucleic acid within a cell, the construct including a promoter specific to the cell, a sequence encoding a Cas nuclease, a promoter enabling expression of a gRNA in the cell, and a sequence encoding a gRNA binding to the target nucleic acid sequence having the insertion or deletion, and
   the construct being configured for the Cas nuclease to produce a cleavage site in the proximity of the target nucleic acid sequence having the insertion or deletion.
(Item B2)
   The construct according to any one of the above items, in which the cleavage site is flanked by a site of the insertion or deletion.
(Item B3)
   The construct according to any one of the above items, in which the condition, disease, disorder, or symptom caused by a frameshift mutation includes retinitis pigmentosa caused by an EYS S1653K frameshift mutation.
(Item B4)
   The construct according to any one of the above items, in which the gRNA binding to the target nucleic acid sequence having the insertion or deletion satisfies a predetermined score in a gRNA design tool.
(Item B4a)
   The construct according to any one of the above items, in which the gRNA binding to the target nucleic acid sequence having the insertion or deletion exhibits a value of a score indicating editing efficiency of about 50% or more in the gRNA design tool.
(Item B4b)
   The construct according to any one of the above items, in which the gRNA binding to the target nucleic acid sequence having the insertion or deletion exhibits a value of a score indicating specificity for the target nucleic acid sequence of about 90% or more in the gRNA design tool.
(Item B4c)
   The construct according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item CC1)
   A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament including:
   a Cas nuclease;
   a gRNA binding to the target nucleic acid sequence having the insertion or deletion; and
   an instruction instructing that the Cas nuclease is administered so as to produce a cleavage site in the proximity of the target nucleic acid sequence having the insertion or deletion.
(Item CC2)
   The medicament according to any one of the above items, in which the medicament is provided in a kit, the kit including:
   the Cas nuclease; and
   the gRNA binding to the target nucleic acid sequence having the insertion or deletion
   in separate compartments.
(Item CC3)
   The medicament according to any one of the above items, in which the medicament is provided as a combination, the combination including:
   the Cas nuclease; and
   the gRNA binding to the target nucleic acid sequence having the insertion or deletion
   in combination.
(Item CC4)
   The medicament according to any one of the above items, in which the medicament is provided as a mixture, the mixture including:
   the Cas nuclease; and
   the gRNA binding to the target nucleic acid sequence having the insertion or deletion
   mixed with each other.
(Item CC5)
   The medicament according to any one of the above items, in which the gRNA binding to the target nucleic acid sequence having the insertion or deletion satisfies a predetermined score in a gRNA design tool.
(Item CC5a)
   The medicament according to any one of the above items, in which the gRNA binding to the target nucleic acid sequence having the insertion or deletion exhibits a value of a score indicating editing efficiency of about 50% or more in the gRNA design tool.
(Item CC5b)
   The medicament according to any one of the above items, in which the gRNA binding to the target nucleic acid sequence having the insertion or deletion exhibits a value of a score indicating specificity for the target nucleic acid sequence of about 90% or more in the gRNA design tool.
(Item CC5c)
   The medicament according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item CD1)
   A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament including:
   a nucleic acid including a nucleic acid sequence encoding a Cas nuclease; and,
   a gRNA binding to the target nucleic acid sequence having the insertion or deletion; and
   an instruction instructing that the Cas nuclease is administered so as to produce a cleavage site in the proximity of the target nucleic acid sequence having the insertion or deletion.
(Item CD2)
   The medicament according to any one of the above items, in which the medicament is provided in a kit, the kit including:
   the nucleic acid including a nucleic acid sequence encoding a Cas nuclease; and
   the gRNA binding to the target nucleic acid sequence having the insertion or deletion
   in separate compartments.
(Item CD3)
   The medicament according to any one of the above items, in which the medicament is provided as a combination, the combination including:
   the nucleic acid including a nucleic acid sequence encoding a Cas nuclease; and
   the gRNA binding to the target nucleic acid sequence having the insertion or deletion
   in combination.
(Item CD4)
   The medicament according to any one of the above items, in which the medicament is provided as a mixture, the mixture including:
   the nucleic acid including a nucleic acid sequence encoding a Cas nuclease; and
   the gRNA binding to the target nucleic acid sequence having the insertion or deletion
   mixed with each other.
(Item CD5)
   The medicament according to any one of the above items, in which the medicament includes a vector, the vector includes a construct including a promoter specific to the cell, a sequence encoding the Cas nuclease, a promoter enabling expression of a gRNA in the cell after introduction of the vector, and a sequence encoding a gRNA binding to the target nucleic acid sequence having the insertion or deletion, and
   the construct is configured for the Cas nuclease to produce a cleavage site in the proximity of the target nucleic acid sequence having the insertion or deletion.
(Item CD6)
   The medicament according to any one of the above items, in which the cleavage site is flanked by a site of the insertion or deletion.
(Item CD7)
   The medicament according to any one of the above items, in which the condition, disease, disorder, or symptom caused by a frameshift mutation includes retinitis pigmentosa caused by an EYS S1653K frameshift mutation.
(Item CD8)
   The medicament according to any one of the above items, in which the gRNA binding to the target nucleic acid sequence having the insertion or deletion satisfies a predetermined score in a gRNA design tool.
(Item CD8a)
   The medicament according to any one of the above items, in which the gRNA binding to the target nucleic acid sequence having the insertion or deletion exhibits a value of a score indicating editing efficiency of about 50% or more in the gRNA design tool.
(Item CD8b)
   The medicament according to any one of the above items, in which the gRNA binding to the target nucleic acid sequence having the insertion or deletion exhibits a value of a score indicating specificity for the target nucleic acid sequence of about 90% or more in the gRNA design tool.
(Item CD8c)
   The medicament according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item CE1)
   A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament including:
   a nucleic acid including a nucleic acid sequence encoding a Cas nuclease;
   a nucleic acid including a nucleic acid sequence of a gRNA binding to the target nucleic acid sequence having the insertion or deletion; and
   an instruction instructing that the Cas nuclease is administered so as to produce a cleavage site in the proximity of the target nucleic acid sequence having the insertion or deletion.
(Item CE2)
   The medicament according to any one of the above items, in which the medicament is provided in a kit, the kit including:
   the nucleic acid including a nucleic acid sequence encoding a Cas nuclease; and
   the nucleic acid including a nucleic acid sequence of a gRNA binding to the target nucleic acid sequence having the insertion or deletion
   in separate compartments.
(Item CE3)
   The medicament according to any one of the above items, in which the medicament is provided as a combination, the combination including:
   the nucleic acid including a nucleic acid sequence encoding a Cas nuclease; and
   the nucleic acid including a nucleic acid sequence of a gRNA binding to the target nucleic acid sequence having the insertion or deletion
   in combination.
(Item CE4)
   The medicament according to any one of the above items, in which the medicament is provided as a mixture, the mixture including:
   the nucleic acid including a nucleic acid sequence encoding a Cas nuclease; and
   the nucleic acid including a nucleic acid sequence of a gRNA binding to the target nucleic acid sequence having the insertion or deletion
   mixed with each other.
(Item CE5)
   The medicament according to the above item, in which the medicament includes a vector, the vector includes a construct including a promoter specific to the cell, a sequence encoding the Cas nuclease, a promoter enabling expression of a gRNA in the cell after introduction of the vector, and a sequence encoding a gRNA binding to the target nucleic acid sequence having the insertion or deletion, and
   the construct is configured for the Cas nuclease to produce a cleavage site in the proximity of the target nucleic acid sequence having the insertion or deletion.
(Item CE6)
   The medicament according to any one of the above items, in which the cleavage site is flanked by a site of the insertion or deletion.
(Item CE7)
   The medicament according to any one of the above items, in which the condition, disease, disorder, or symptom caused by a frameshift mutation includes retinitis pigmentosa caused by an EYS S1653K frameshift mutation.
(Item CE8)
   The medicament according to any one of the above items, in which the gRNA binding to the target nucleic acid sequence having the insertion or deletion satisfies a predetermined score in a gRNA design tool.
(Item CE8a)
   The medicament according to any one of the above items, in which the gRNA binding to the target nucleic acid sequence having the insertion or deletion exhibits a value of a score indicating editing efficiency of about 50% or more in the gRNA design tool.
(Item CE8b)
   The medicament according to any one of the above items, in which the gRNA binding to the target nucleic acid sequence having the insertion or deletion exhibits a value of a score indicating specificity for the target nucleic acid sequence of about 90% or more in the gRNA design tool.
(Item CE8c)
   The medicament according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item CF1)
   A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament including:
   a Cas nuclease;
   a nucleic acid including a nucleic acid sequence of a gRNA binding to the target nucleic acid sequence having the insertion or deletion; and
   an instruction instructing that the Cas nuclease is administered so as to produce a cleavage site in the proximity of the target nucleic acid sequence having the insertion or deletion.
(Item CF2)
   The medicament according to any one of the above items, in which the medicament is provided in a kit, the kit including:
   the Cas nuclease; and
   the nucleic acid including a nucleic acid sequence of a gRNA binding to the target nucleic acid sequence having the insertion or deletion
   in separate compartments.
(Item CF3)
   The medicament according to any one of the above items, in which the medicament is provided as a combination, the combination including:
   the Cas nuclease; and
   the nucleic acid including a nucleic acid sequence of a gRNA binding to the target nucleic acid sequence having the insertion or deletion
   in combination.
(Item CF4)
   The medicament according to any one of the above items, in which the medicament is provided as a mixture, the mixture including:
   the Cas nuclease; and
   the nucleic acid including a nucleic acid sequence of a gRNA binding to the target nucleic acid sequence having the insertion or deletion
   mixed with each other.
(Item CF5)
   The medicament according to the above item, in which the medicament includes a vector, the vector includes a construct including a promoter specific to the cell, a sequence encoding the Cas nuclease, a promoter enabling expression of a gRNA in the cell after introduction of the vector, and a sequence encoding a gRNA binding to the target nucleic acid sequence having the insertion or deletion, and
   the construct is configured for the Cas nuclease to produce a cleavage site in the proximity of the target nucleic acid sequence having the insertion or deletion.
(Item CF6)
   The medicament according to any one of the above items, in which the cleavage site is flanked by a site of the insertion or deletion.
(Item CF7)
   The medicament according to any one of the above items, in which the condition, disease, disorder, or symptom caused by a frameshift mutation includes retinitis pigmentosa caused by an EYS S1653K frameshift mutation.
(Item CF8)
   The medicament according to any one of the above items, in which the gRNA binding to the target nucleic acid sequence having the insertion or deletion satisfies a predetermined score in a gRNA design tool.
(Item CF8a)
   The medicament according to any one of the above items, in which the gRNA binding to the target nucleic acid sequence having the insertion or deletion exhibits a value of a score indicating editing efficiency of about 50% or more in the gRNA design tool.
(Item CF8b)
   The medicament according to any one of the above items, in which the gRNA binding to the target nucleic acid sequence having the insertion or deletion exhibits a value of a score indicating specificity for the target nucleic acid sequence of about 90% or more in the gRNA design tool.
(Item CF8c)
   The medicament according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item C1)
   A method for altering, preventing, or treating a condition, disease, disorder, or symptom caused by a frameshift mutation due to insertion or deletion of one or more bases by cleaving at least one site of a target nucleic acid sequence in a nucleic acid within a cell, the method including:
   introducing a vector into the cell, the vector including a construct including a promoter specific to the cell, a sequence encoding the Cas nuclease, a promoter enabling expression of a gRNA in the cell after introduction of the vector, and a sequence encoding a gRNA binding to the target nucleic acid sequence having the insertion or deletion; and
   placing the cell in a condition under which the cleavage occurs,
   the construct being configured for the Cas nuclease to produce a cleavage site in the proximity of the target nucleic acid sequence having the insertion or deletion.
(Item C2)
   The method according to any one of the above items, in which the cleavage site is flanked by a site of the insertion or deletion.
(Item C3)
   The method according to any one of the above items, in which the condition, disease, disorder, or symptom caused by a frameshift mutation includes retinitis pigmentosa caused by an EYS S1653K frameshift mutation.
(Item C4)
   The method according to any one of the above items, in which the vector includes an adeno-associated virus (AAV) vector.
(Item C5)
   The method according to any one of the above items, in which the gRNA binding to the target nucleic acid sequence having the insertion or deletion satisfies a predetermined score in a gRNA design tool.
(Item C5a)
   The method according to any one of the above items, in which the gRNA binding to the target nucleic acid sequence having the insertion or deletion exhibits a value of a score indicating editing efficiency of about 50% or more in the gRNA design tool.
(Item C5b)
   The method according to any one of the above items, in which the gRNA binding to the target nucleic acid sequence having the insertion or deletion exhibits a value of a score indicating specificity for the target nucleic acid sequence of about 90% or more in the gRNA design tool.
(Item C5c)
   The method according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item D1)
   A method for producing a product for altering, preventing, or treating a condition, disease, disorder, or symptom by cleaving at least one site of a target nucleic acid sequence in a nucleic acid within a cell, the method including:
   detecting a frameshift mutation due to insertion or deletion of one or more bases causing the condition, disease, disorder, or symptom;
   designing a construct based on the frameshift mutation, the construct including a promoter specific to the cell, a sequence encoding a Cas nuclease, a promoter enabling expression of a gRNA in the cell after introduction of the vector, and a sequence encoding a gRNA binding to the target nucleic acid sequence having the insertion or deletion;
   producing and inserting the construct into the vector; and
   producing a product including the vector,
   the construct being configured for the Cas nuclease to produce a cleavage site in the proximity of the target nucleic acid sequence having the insertion or deletion.
(Item D2)
   The method according to any one of the above items, in which the cleavage site is flanked by a site of the insertion or deletion.
(Item D3)
   The method according to any one of the above items, in which the vector includes an adeno-associated virus (AAV) vector.
(Item D4)
   The method according to any one of the above items, in which the gRNA binding to the target nucleic acid sequence having the insertion or deletion satisfies a predetermined score in a gRNA design tool.
(Item D4a)
   The method according to any one of the above items, in which the gRNA binding to the target nucleic acid sequence having the insertion or deletion exhibits a value of a score indicating editing efficiency of about 50% or more in the gRNA design tool.
(Item D4b)
   The method according to any one of the above items, in which the gRNA binding to the target nucleic acid sequence having the insertion or deletion exhibits a value of a score indicating specificity for the target nucleic acid sequence of about 90% or more in the gRNA design tool.
(Item D4c)
   The method according to any one of the above items, in which the gRNA binding to the target nucleic acid sequence having the insertion or deletion exhibits a value of an MIT Specificity Score of about 30% or more in CRISPOR or a corresponding value thereto or more in another design tool.
(Item EA1)
   A method for altering, preventing, or treating a condition, disease, disorder, or symptom caused by a frameshift mutation due to insertion or deletion of one or more bases in a patient by cleaving at least one site of a target nucleic acid sequence in a nucleic acid within a cell, the method including:
   sequencing the target nucleic acid sequence in a nucleic acid derived from the patient;
   selecting a gRNA that matches the target nucleic acid sequence in the patient based on the sequencing;
   introducing a vector into the cell, the vector including a construct including a promoter specific to the cell, a sequence encoding the Cas nuclease, a promoter enabling expression of a gRNA in the cell after introduction of the vector, and a sequence encoding a gRNA binding to the target nucleic acid sequence having the insertion or deletion; and
   placing the cell in a condition under which the cleavage occurs,
   the construct being configured for the Cas nuclease to produce a cleavage site in the proximity of the target nucleic acid sequence having the insertion or deletion.
(Item EA2)
   The method according to the above item, further including one or more features according to the above item.
(Item EB1)
   Use of a vector for producing a medicament for altering, preventing, or treating a condition, disease, disorder, or symptom caused by a frameshift mutation due to insertion or deletion of one or more bases in a patient by cleaving at least one site of a target nucleic acid sequence in a nucleic acid within a cell, the vector including a construct that includes a promoter specific to the cell, a sequence encoding a Cas nuclease, a promoter enabling expression of a gRNA in the cell after introduction of the vector, and a sequence encoding a gRNA binding to the target nucleic acid sequence having the insertion or deletion, the altering, preventing, or treating being achieved by a method including:
   sequencing the target nucleic acid sequence in a nucleic acid derived from the patient;
   selecting a gRNA that matches the target nucleic acid sequence in the patient based on the sequencing;
   introducing the vector into the cell; and
   placing the cell in a condition under which the cleavage occurs,
   the construct being configured for the Cas nuclease to produce a cleavage site in the proximity of the target nucleic acid sequence having the insertion or deletion.
(Item EB2)
   The use according to the above item, further including one or more features according to the above item.
(Item EC1)
   A medicament for altering, preventing, or treating a condition, disease, disorder, or symptom caused by a frameshift mutation due to insertion or deletion of one or more bases in a patient by cleaving at least one site of a target nucleic acid sequence in a nucleic acid within a cell, the medicament including:
   a Cas nuclease;
   a gRNA binding to the target nucleic acid sequence having the insertion or deletion; and
   an instruction instructing that the Cas nuclease is administered so as to produce a cleavage site in the proximity of the target nucleic acid sequence having the insertion or deletion.
   the altering, preventing, or treating being achieved by a method including:
      sequencing the target nucleic acid sequence in a nucleic acid derived from the patient;
      selecting a gRNA that matches the target nucleic acid sequence in the patient based on the sequencing;
      introducing a vector into the cell, the vector including a construct including a promoter specific to the cell, a sequence encoding the Cas nuclease, a promoter enabling expression of a gRNA in the cell after introduction of the vector, and a sequence encoding a gRNA binding to the target nucleic acid sequence having the insertion or deletion; and
      placing the cell in a condition under which the cleavage occurs,
      the construct being configured for the Cas nuclease to produce a cleavage site in the proximity of the target nucleic acid sequence having the insertion or deletion.
(Item EC2)
   The medicament according to the above item, further including one or more features according to the above item.

In the present disclosure, it is intended that the above-mentioned one or more features may be provided in further combinations in addition to the explicit combinations. Note that, those skilled in the art will be recognized further embodiments and advantages of the present disclosure upon reading and understanding the following detailed description, if necessary.

Note that, features and significant actions and effects of the present disclosure other than those described above will become clear to those skilled in the art by referring to the following embodiments of the invention and drawings.

### [Advantageous Effects of Invention]

According to the present disclosure, a gene that has undergone a frameshift mutation can be returned to a normal sequence or a sequence that is similar thereto by targeting the target nucleic acid sequence in a genome within a cell, which can be widely applied to treat various hereditary diseases or to correct many mutations.

### [Brief Description of Drawings]

[FIG. 1] Figure 1 is a schematic diagram showing a design of a gene therapy according to one embodiment of the present disclosure targeting an etiologic mutation in EYS which is a gene responsible for retinitis pigmentosa;
[Figure 2] Figure 2 is a table and graph showing results of sequencing by a MiSeq sequencer in one embodiment of the present disclosure. Figure 2a shows a table of representative data analyzed by CRISPECTOR. Figure 2b shows on-target sequencing results of a genomic DNA obtained from an LCL cell line into which a genome editing vector had been introduced. As a control, data on MMEJ (microhomology-mediated end-joining) obtained in another mouse model (Nishiguchi et al. Nat Commun. 2020; 11(1):482) are shown;
[Figure 3] Figure 3 shows results of comparison of editing efficiencies when a method according to one embodiment of the present disclosure and an MMEJ method were used. Figure 3a is a graph showing results of on-target sequencing with a MinION nanopore sequencer of a genomic DNA obtained from a lymphoblast cell line (LCL) after introduction of a genome editing vector. Figure 3b is electrophoresis images showing results of RT-PCR analysis of an EYS mRNA obtained from a HEK293T stabilized cell line after introduction of a genome editing vector;
[Figure 4] Figure 4 is a schematic diagram showing cleavage sites of a GJB2 gene when the GJB2 gene is cleaved using a medicament according to one embodiment of the present disclosure for treating hereditary hearing loss caused by a mutated GJB2 gene;

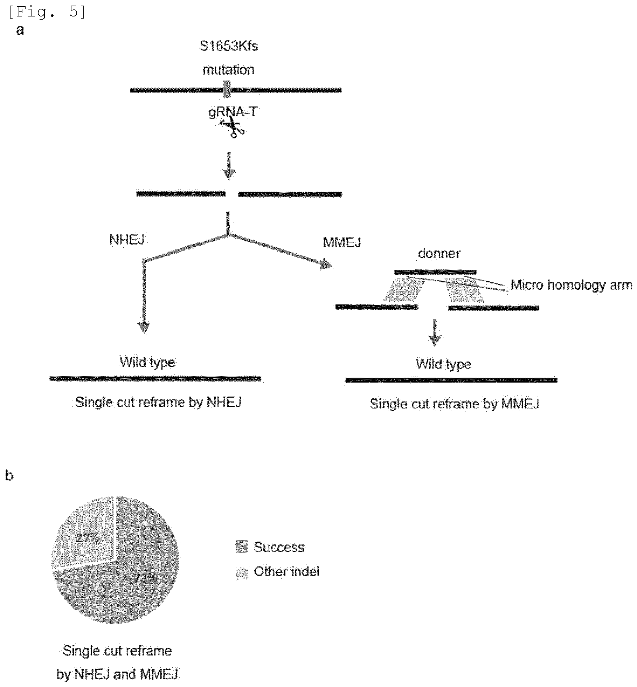

[Figure 5] Figure 5 is a schematic diagram and graph showing results of in vitro evaluation on an on-target site for repair by NHEJ (non-homologous end joining) and MMEJ in one embodiment of the present disclosure. Figure 5a is a schematic diagram of a repair strategy for a frameshift mutation via both the NHEJ and the MMEJ. The frameshift mutation in an edited site is repaired by insertion of a normal sequence by the NHEJ or the MMEJ. Figure 5b shows results of on-target sequencing by a MiSeq sequencer of a genomic DNA obtained from an LCL cell line into which a genome editing vector had been introduced;
[Figure 6] Figure 6 is a schematic diagram showing cleavage sites of a FOXG1 gene when the FOXG1 gene is cleaved using a medicament according to one embodiment of the present disclosure for treating Rett syndrome caused by a mutated FOXG1 gene;
[Figure 7] Figure 7 is a schematic diagram showing a cleavage site of a CHAMP1 gene when the CHAMP1 gene is cleaved using a medicament according to one embodiment of the present disclosure for treating intellectual disability caused by a mutated CHAMP1 gene;
[Figure 8] Figure 8 is a schematic diagram showing a cleavage site of a SEPN1 gene when the SEPN1 gene is cleaved using a medicament according to one embodiment of the present disclosure for treating SELENON-related congenital myopathy caused by a mutated SEPN1 gene;
[Figure 9] Figure 9 is a schematic diagram showing a cleavage site of an HSPB8 gene when the HSPB8 gene is cleaved using a medicament according to one embodiment of the present disclosure for treating inclusion body myopathy caused by a mutated HSPB8 gene;
[Figure 10] Figure 10 is a schematic diagram showing a cleavage site of an NGLY1 gene when the NGLY1 gene is cleaved using a medicament according to one embodiment of the present disclosure for treating congenital disorder of deglycosylation caused by a mutated NGLY1 gene;
[Figure 11] Figure 11 is a diagram showing in vitro evaluation on an on-target site of an NGLY1 c.1370dupG mutation by MiSeq sequencing. Figure 11a shows a table of representative data analyzed by CRISPECTOR. Figure 11b is a graph showing results of on-target sequencing of a genomic DNA obtained from a fibroblast cell line after introduction of a genome editing vector;
[Figure 12] Figure 12 is a diagram showing in vitro evaluation on an on-target site of a USH1C 238insC mutation by MiSeq sequencing. Figure 12a shows a schematic diagram of a designed gRNA. Figure 12b shows a table of representative data analyzed by CRISPECTOR. Figure 12c is a graph showing results of on-target sequencing of a genomic DNA obtained from a fibroblast cell line after introduction of a genome editing vector;
[Figure 13] Figure 13 is a diagram showing in vitro evaluation on an on-target site of a FOXG1 c.460dupG mutation by MiSeq sequencing. Figure 13a shows a table of representative data analyzed by RISPECTOR. Figure 13b is a graph showing results of on-target sequencing of a genomic DNA obtained from a fibroblast cell line after introduction of a genome editing vector;
[Figure 14] Figure 14 is a diagram showing in vitro evaluation on an on-target site of an Lpcat1 rd11 mutation by MiSeq sequencing. Figure 14a shows a table of representative data analyzed by CRISPECTOR. Figure 14b is a graph showing results of on-target sequencing of a genomic DNA obtained from a fibroblast cell line after introduction of a genome editing vector;
[Figure 15] Figure 15 is a schematic diagram showing a cleavage site of a PEX26 gene when the PEX26 gene is cleaved using a medicament according to one embodiment of the present disclosure for treating Zellweger syndrome caused by a mutation on the PEX26 gene;
[Figure 16] Figure 16 is a schematic diagram showing a cleavage site of a SHOX gene when the SHOX gene is cleaved using a medicament according to one embodiment of the present disclosure for treating Langer mesomelic dysplasia caused by a mutation on the SHOX gene;
[Figure 17] Figure 17 is a schematic diagram showing a cleavage site of a BRAT1 gene when the BRAT1 gene is cleaved using a medicament according to one embodiment of the present disclosure for treating rigidity and multifocal seizure syndrome caused by a mutation on the BRAT1 gene;
[Figure 18] Figure 18 is a schematic diagram showing a cleavage site of a GNPTAB gene when the GNPTAB gene is cleaved using a medicament according to one embodiment of the present disclosure for treating mucolipidosis II caused by a mutation on the GNPTAB gene;
[Figure 19] Figure 19 is a schematic diagram showing a cleavage site of an NR0B1 gene when the NR0B1 gene is cleaved using a medicament according to one embodiment of the present disclosure for treating congenital adrenal hyperplasia (CAH) caused by a mutation on the NR0B1 gene;
[Figure 20] Figure 20 is a schematic diagram showing a cleavage site of a POLH gene when the POLH gene is cleaved using a medicament according to one embodiment of the present disclosure for treating xeroderma pigmentosum caused by a mutation on the POLH gene;
[Figure 21] Figure 21 is a schematic diagram showing a cleavage site of a GAA gene when the GAA gene is cleaved using a medicament according to one embodiment of the present disclosure for treating glycogen storage disease type II caused by a mutation on the GAA gene;
[Figure 22] Figure 22 is a schematic diagram showing a cleavage site of an NPC1 gene when the NPC1 gene is cleaved using a medicament according to one embodiment of the present disclosure for treating Niemann-Pick disease caused by a mutation on the NPC1 gene; and
[Figure 23] Figure 23 is a schematic diagram showing a cleavage site of a USH2A gene when the USH2A gene is cleaved using a medicament according to one embodiment of the present disclosure for treating Usher syndrome caused by a mutation on the USH2A gene.

### [Description of Embodiments]

The present disclosure will be described with reference to the best mode thereof. It should be understood that throughout this specification, singular expressions also include the concept of their plural forms, unless otherwise noted. Thus, it should be understood that singular articles (e.g., "a", "an", "the", etc. in English) also include the concept of their plural forms, unless otherwise noted. It should also be understood that terms used herein are to be used in the sense normally used in the art, unless otherwise noted. Therefore, unless otherwise defined, all technical and scientific terms used herein have the same meaning as commonly understood by those skilled in the art to which this disclosure pertains. In the event of a conflict, this specification (including definitions) shall prevail.

Definitions and/or basic technical details of terms specifically used herein will be described as appropriate.

As used herein, the term "about" means ±10% of the numeral value that follows.

As used herein, the term "genome" refers to genetic information of an organism. The phrase "nucleic acid genome" refers to a genome that is a nucleic acid (especially DNA or RNA) and is used interchangeably with "genomic nucleic acid" herein.

As used herein, the term "modification" in the context of a gene sequence means insertion of a base sequence (which in this context may be at least one base) into a nucleic acid (e.g., DNA) in a cell, deletion of a base sequence (which in this context may be at least one base) in a nucleic acid (e.g., DNA) in a cell, or substitution (replacement) of a base sequence (which in this context may be at least one base) of a nucleic acid (e.g., DNA) in a cell with another base sequence, or a combination thereof.

As used herein, a cleavage site that is present "around a target nucleic acid sequence" means not only a cleavage site that is present on the target nucleic acid sequence, but also a cleavage site that is upstream or downstream of a nucleic acid sequence of interest.

As used herein, a cleavage site that is present "in the proximity of a target nucleic acid sequence" means not only a cleavage site that is present on the target nucleic acid sequence, but also the cleavage site that is present within about 20 bases upstream or downstream a nucleic acid sequence of interest.

As used herein, the cleavage site that is "flanked by a target nucleic acid sequence" means a cleavage site that is present between a nucleic acid sequence of interest and a nucleic acid that is contiguously present upstream or downstream of the nucleic acid sequence.

As used herein, "cleaved so that a desired frameshift recovery is achieved as a result of cleavage" means a cleavage so that, as a result of cleavage of a target nucleic acid sequence by a Cas nuclease, a frameshift in the target nucleic acid sequence returns to its original state and a condition, disease, disorder, or symptom caused by the frameshift mutation is treated or the like.

As used herein, the term "alter(ing)" in the context of a condition, disease, disorder, or symptom means producing any change in the condition, disease, disorder, or symptom including ameliorating the condition, disease, disorder, or symptom and exacerbating the condition, disease, disorder, or symptom.

As used herein, the term "treatment" means, either prophylactic and/or therapeutic in the broad sense, or for the purpose of improvement (cure) from a morbid condition in the narrow sense, alleviating, weakening, or ameliorating at least one symptom of a disease or condition, preventing an additional symptom, inhibiting a disease or condition, for example, suppressing an onset of a disease or condition, mitigating a disease or condition, causing a regression of a disease or condition, mitigating a condition caused by a disease or condition, or arresting a symptom of a disease or condition. As used herein, the term "treatment" means alleviating, weakening, or ameliorating at least one symptom of a disease or condition for the purpose of improvement (cure) from a morbid condition.

As used herein, the term "prevention" means preventing a clinical symptom of a disease state from developing in a subject who is at risk of exposure or susceptibility to the disease state, but who has not yet experienced or developed a symptom of the disease state.

As used herein, the phrase "frameshift mutation" refers to a mutation caused by deletion or insertion of one or more nucleotides in an open reading frame such that a reading frame of a coding region is shifted by one or two nucleotides. Thus, an amino acid sequence of a polypeptide translated from an mRNA carrying the frameshift mutation is different from a corresponding wild-type sequence. Deletion or insertion of any number of nucleotides may occur herein as long as it results in the frameshift mutation. Another genetic modification that is responsible for frameshift is also encompassed by the "frameshift mutation" herein. For example, exon skipping, retention of an intron sequence, or a change in a nucleotide sequence that results in translation initiation from a different position is also encompassed by the "frameshift mutations" herein. The frameshift mutation herein may result in a premature termination codon and includes one that results in an amino acid sequence differing from a wild-type sequence.

As used herein, the term "gene" refers to a factor that determines a genetic trait, and the "gene" may be a nucleic acid itself or may refer to a "polynucleotide", an "oligonucleotide", an "RNA", or a "DNA", and also sometimes refers to a protein, polypeptide, oligopeptide or peptide encoded by a nucleic acid, which can be appropriately understood by those skilled in the art according to the context. A gene encoding such a protein may be endogenous or exogenous to an organism of interest. Such known genes can also be used as appropriate. The gene can be used regardless of an organism from which the gene is derived. That is, the gene may be derived from an organism of another species or genus other than an organism of interest, or may be derived from an organism such as an animal, a plant, a fungus (such as a mold), bacterium, or the like. Those skilled in the art can obtain information on such a gene by accessing a website such as the NCBI (National Center for Biotechnology Information; http://www.ncbi.nlm.nih.gov) as appropriate. Such a gene may be a gene that encodes a protein having a certain relationship to sequence information disclosed in a database, etc., as long as it exhibits an activity that the protein has.

The terms "protein", "polypeptide", "oligopeptide", and "peptide" are used herein in the same meaning and refer to a polymer of amino acids having any length. The polymer may be linear, branched, or cyclic. The amino acid may be a naturally occurring, non-naturally occurring, or modified amino acid. The terms can also encompass multiple polypeptide chains assembled into a complex. The terms also encompass a polymer of naturally occurring or artificially modified amino acids. Examples of such a modification include, for example, formation of a disulfide bond, glycosylation, lipidation, acetylation, phosphorylation, or any other manipulation or modification (e.g., conjugation with a labeled component). This definition also encompasses, for example, a polypeptide containing one or two or more amino acid analogs (including, for example, non-naturally occurring amino acids), a peptide-like compound (e.g., peptoid), and other modifications known in the art. As used herein, the phrase "amino acid" is a generic term for organic compounds each having an amino group and a carboxyl group. When an antibody according to an embodiment of the present disclosure contains a "specific amino acid sequence", any of amino acids in the amino acid sequence may be chemically modified. Any of the amino acids in the amino acid sequence may also form a salt or a solvate. In addition, any of the amino acids in the amino acid sequence may be L- or D-type. Even in those cases, a protein according to the embodiment of the present disclosure can be said to include the above "specific amino acid sequence". Examples of known chemical modifications that an amino acid included in a protein undergoes in vivo include an N-terminal modification (e.g., acetylation, myristoylation, etc.), a C-terminal modification (e.g., amidation, glycosylphosphatidylinositol addition, etc.), or a side chain modification (e.g., phosphorylation, glycosylation, etc.). The amino acid may or may not be naturally occurring as long as it achieves the purpose of the present disclosure.

As used herein, the terms "polynucleotide", "oligonucleotide", and "nucleic acid" are used herein in the same meaning, refer to a polymer of nucleotides having any length, and include DNA and RNA. These terms also include an "oligonucleotide derivative" or a "polynucleotide derivative". The terms "oligonucleotide derivative" and "polynucleotide derivative" refer to an oligonucleotide or polynucleotide that includes a nucleotide derivative or has an unusual bond between nucleotides and are used interchangeably. Specific examples of such an oligonucleotide include a 2'-O-methyl-ribonucleotide, an oligonucleotide derivative in which a phosphodiester bond in an oligonucleotide is converted to a phosphorothioate bond, an oligonucleotide derivative in which a phosphate diester bond in an oligonucleotide is converted to an N3'-P5' phosphoramidate bond, an oligonucleotide derivative in which a ribose-phosphate diester linkage in an oligonucleotide is converted to a peptide-nucleic acid linkage, an oligonucleotide derivative in which a uracil in an oligonucleotide is replaced with a C-5 propynyl uracil, an oligonucleotide derivative in which a uracil in an oligonucleotide is replaced with a C-5 thiazole uracil, an oligonucleotide derivative in which a cytosine in an oligonucleotide is replaced with a C-5 propynyl cytosine, an oligonucleotide derivative in which a cytosine in an oligonucleotide is replaced with a phenoxazine-modified cytosine, an oligonucleotide derivative in which a ribose in DNA is replaced with a 2'-O-propylribose, and an oligonucleotide derivative in which a ribose in an oligonucleotide is replaced with a 2'-methoxyethoxyribose. Unless otherwise indicated, it is contemplated that a specific nucleic acid sequence will also encompass an explicit sequence as well as its conservatively modified modification (e.g., condensed codon substitution) and complementary sequences thereof. Specifically, the condensed codon substitution can be achieved by creating a sequence in which the third positions of one or more selected (or all) codons are replaced with mixed bases and/or deoxyinosine residues (Batzer et al., Nucleic Acid Res.19:5081 (1991); Ohtsuka et al., J. Biol. Chem. 260:2605-2608 (1985); Rossolini et al., Mol. Cell. Probes 8:91-98 (1994)). As used herein, the term "nucleic acid" is used interchangeably with the terms gene, cDNA, mRNA, oligonucleotide, and polynucleotide. As used herein, the term "nucleotide" may refer to a naturally occurring nucleotide or a non-naturally occurring nucleotide.

An amino acid may be referred to herein either by its commonly known three-letter code or by one-letter code recommended by the IUPAC-IUB Biochemical Nomenclature Commission. Similarly, a nucleotide can also be referred to by a commonly recognized one-letter code. Comparison of similarity, identity, and homology between amino acid sequences or base sequences are herein calculated using default parameters in BLAST, a tool for sequence analysis. An identity search can be performed, for example, using BLAST 2.2.28 by the NCBI (published April 2, 2013). A value of identity herein usually refers to a value determined when aligned under default conditions using the BLAST. However, if a change in parameters results in a higher value, the highest value shall be a value of identity. When identity is evaluated in a plurality of regions, the highest value shall be a value of identity. Similarity refers to a numerical value calculated taking similar amino acids into account in addition to identity.

In one embodiment of the present disclosure, "90% or more" may be, for example, 90, 95, 96, 97, 98, 99, or 100% or more, or may be in a range between any two of these values. "Homology" may be calculated as a percentage of homologous amino acids between two or more amino acid sequences according to a method known in the art. Before calculating the percentage, amino acid sequences in amino acid sequence groups to be compared are aligned, and a gap is introduced in a portion of the amino acid sequences if necessary to maximize a percentage of identical amino acids. Methods for alignment, calculation of the percentage, and comparison, and computer programs related thereto are conventionally well-known in the art (e.g., BLAST, GENETYX, etc.). As used herein, the term "homology" can be expressed as a value determined using BLAST by the NCBI, unless otherwise noted. Blastp can be used with default setting as an algorithm when comparing amino acid sequences by BLAST. Measurement results are converted into numbers as Positives or Identities.

As used herein, the term "vector" means a nucleic acid sequence containing an origin of replication. The vector may be a viral vector, a bacteriophage, a bacterial artificial chromosome, or a yeast artificial chromosome. The vector may be a DNA vector or an RNA vector. The vector may be a self-replicating extrachromosomal vector, preferably a DNA plasmid.

As used herein, the term "construct" refers to one or more nucleic acids having a certain structure, which is naturally occurring and/or non-naturally occurring. Unless explicitly specified, the "construct" refers to one or more constructs. The construct is not limited to one that itself is isolated, but when a foreign sequence is inserted into a longer nucleic acid **(e.g.,** genomic DNA), it may also refer to a portion having a non-naturally occurring structure that contains the foreign sequence.

As used herein, the phrase "adeno-associated virus (AAV) vector" refers to a transcription regulatory element, **i.e., a** protein coding sequence operatively linked to one or more promoters and/or enhancers, and a polyadenylation sequence, and, if desired, a single-stranded or double-stranded nucleic acid having an AAV **5'** inverted terminal repeat (ITR) sequence and an AAV **3'** ITR flanked by one or more introns inserted between exons of the protein coding sequence. A single-stranded AAV vector refers to a nucleic acid that is present in a genome of AAV virus particles and may be either a sense or antisense strand of a nucleic acid sequence disclosed herein. A size of such a single-stranded nucleic acid is given in a base unit. A double-stranded AAV vector refers to a nucleic acid that is present in a DNA of a plasmid **(e.g.,** pUC19) to be used for expression or gene transfer of a nucleic acid of an AAV vector or in a genome of a double-stranded virus **(e.g.,** baculovirus). A size of such a double-stranded nucleic acid is given in a base pair (bp) unit.

The terms "guide RNA", "gRNA", "single-stranded gRNA", and "sgRNA", which are used interchangeably herein, refer to a short synthetic RNA that contains at least a spacer sequence that hybridizes with a target nucleic acid sequence of interest, and a CRISPR repeat sequence.

As used herein, the phrase "gRNA target sequence" refers to a sequence targeted by a guide RNA.

As used herein, the phrase "target nucleic acid sequence" refers to any nucleotide sequence that encodes a known or putative gene product. The target nucleic acid sequence can be a mutated gene involved in a hereditary disease.

As used herein, the phrase "gRNA binding to a target nucleic acid sequence having insertion or deletion" refers to a gRNA binding to a target nucleic acid sequence having insertion or deletion among the above-described target nucleic acid sequences.

As used herein, the term "promoter specific to a cell" refers to a promoter that specifically functions in a given cell, for example, examples of those to be used in the retina include a rhodopsin kinase promoter, an RPE65 promoter, a Best1 promoter, an mGluR6 promoter, a cone arrestin promoter, a CRALBP1 promoter, a Chx10 promoter, a rhodopsin promoter, a cone opsin promoter, and a recoverin promoter.

As used herein, the phrase "promoter enabling expression of a gRNA" can be any promoter that enables gRNA expression, and includes, for example, an RNA polymerase III promoter such as a U6 promoter, an H1 promoter, and a 7SK promoter.

As used herein, the term "Cas", "Cas protein", or "Cas nuclease" refers to an RNA-induced nuclease including a Cas protein (e.g., Cas nuclease from diverse bacterial species), a fragment or variant thereof (e.g., catalytically inactive Cas or Cas nickase), or a fusion protein (e.g., Cas fused to another protein domain). For example, those having the same function as Cas9 are encompassed by the scope of the present disclosure even if the term Cas9 is not used in the name. Examples of the Cas can include Cas9 and Cas12.

As used herein, the term "introduction" of a gene or nucleic acid refers to introduction of an exogenous or endogenous gene, preferably a functional gene, into, for example, a chromosomal genome by means of an appropriate introduction technique. A gene can be introduced using a vector such as a phage and a plasmid, and a spontaneous transformation method, a conjugation method, a protoplast-PEG method, and an electroporation method can also be used. Also, when using a target gene recombination method known in the art, an exogenous functional gene can be introduced by replacing an endogenous functional gene with it. Note that, the exogenous functional gene is a gene that is not originally present in a chromosomal genome of an organism, and may be a gene derived from another species or a synthetic gene created by, for example, PCR. Introduction of a gene also encompasses conversion of a gene to a desired gene by genome editing on an existing genome.

As used herein, the phrase "flanked by" a sequence relates to a positional relationship either upstream or downstream of the sequence. For example, a sequence B flanked by a sequence A means the sequence B located adjacent upstream or downstream to the sequence A, and it is understood that the sequence B that is present in the proximity of the sequence A to a certain degree is considered to be "flanked by" the sequence A even if not directly adjacent to the sequence A, as long as the purpose of the present disclosure is achieved.

As used herein, the phrase "PAM sequence" or "protospacer adjacent motif" is used interchangeably with each other and refers to a sequence that is recognized by a Cas protein upon DNA cleavage by the Cas protein. A sequence and location of the PAM vary with a type of the Cas protein. For example, in the case of a Cas9 protein, the PAM must be immediately flanked by a 3' side of a target sequence. A sequence of PAM corresponding to the Cas9 protein varies with a bacterial species from which the Cas9 protein is derived. For example, a PAM corresponding to a Cas9 protein from S. pyogenes is "NGG", a PAM corresponding to a Cas9 protein from S. thermophilus is "NNAGAA", a PAM corresponding to a Cas9 protein from S. aureus is "NNGRRT" or "NNGRR (N)", a PAM corresponding to a Cas9 protein from N. meningitidis is "NNNNGATT", and a PAM corresponding to a Cas9 protein from T. denticola is "NAAAAC" (where "R" is A or G; "N" is A, T, G or C).

As used herein, the term "subject" includes, for example, human; non-human mammals such as cattle, pig, sheep, goat, rabbit, dog, cat, guinea pig, hamster, mouse, rat, and monkey; birds; fishes such as zebrafish; amphibians such as frog; reptiles; insects such as Drosophila; crustaceans, etc.

As used herein, the phrase "condition, disease, disorder, or symptom" means those that can be treated or prevented using a technique of the present disclosure, and, for example, encompasses an ocular disease such as a hereditary retinal degenerative disease, retinitis pigmentosa, macular degeneration, glaucoma, cataract, uveitis, optic neuritis, diabetic retinopathy, a retinal vascular occlusive disease, age-related macular degeneration, corneal dystrophy, bullous keratopathy, or corneal opacity; as well as hereditary deafness, Parkinson's disease, Huntington's disease, Alzheimer's disease, multiple sclerosis, rheumatoid arthritis, Crohn's disease, Peyronie's disease, ulcerative colitis, cerebral ischemia (stroke), myocardial infarction (heart attack), brain damage and/or spinal cord damage, reperfusion injury, ALS, Down's syndrome, cataract, schizophrenia, epilepsy, human leukemia and other cancers, and diabetes mellitus, and similar or any other hereditary diseases.

As used herein, the phrase "ocular disease" refers to a disease related to the eye and includes a disease that causes a symptom in the eye. Various diseases that are expected to be improved by gene manipulation or editing are particularly preferred in the present disclosure, and examples thereof include a hereditary retinal disease (retinitis pigmentosa, Leber's congenital blindness, rod-cone dystrophy, cone dystrophy, macular dystrophy, achromatopsia, retinal dystrophy, etc.), a non-hereditary retinal disease (age-related macular degeneration, diabetic retinopathy, retinal vein occlusion, retinal artery occlusion, myopic retinopathy, retinopathy of prematurity, choroidal neovascularization, central chorioretinopathy, etc.), an optic nerve disease (hereditary optic atrophy, Leber's optic neuropathy, traumatic optic neuropathy, optic neuritis, glaucoma, etc.), uveitis (Harada disease, Behcet's disease, sarcoidosis, panuveitis, posterior uveitis, intermediate uveitis, anterior uveitis, etc.), an ocular infection (cytomegalovirus infection, herpes infection, etc.), an ocular tumor (malignant lymphoma, etc.), a corneal disease (corneal dystrophy, dry eye, bullous keratopathy, corneal opacity, etc.), myopia, hyperopia, astigmatism, amblyopia, etc.

As used herein, the phrase "hereditary disease" refers to a disease, especially an innate condition, that is partially or completely and directly or indirectly caused by one or more aberrations in a genome. The aberration may be a mutation, insertion, or deletion. The aberration may affect a coding sequence of a gene or its regulatory sequence. The hereditary disease includes, but is not limited to, DMD, hemophilia, cystic fibrosis, Huntington's disease, familial hypercholesterolemia (LDL receptor deficiency), hepatoblastoma, Wilson's disease, congenital hepatic porphyria, a hereditary disorder of liver metabolism, Lesch-Nyhan syndrome, sickle cell anemia, Mediterranean anemia, xeroderma pigmentosum, Fanconi anemia, retinitis pigmentosa, ataxia telangiectasia, Bloom's syndrome, retinoblastoma, and Tay-Sachs disease.

As used herein, the phrase "genome editing" refers to alteration of a gene. The genome editing can include correcting or repairing a mutated gene. The genome editing can include knocking out a gene such as a mutated or normal gene. The genome editing can be used to treat a disease by altering a gene of interest.

As used herein, the phrase "CRISPR-Cas" or "CRISPR system" collectively refers to a transcript and other sequence elements involved in development or induction of an activity of a CRISPR-related ("Cas") gene, as described in, for example, WO2014/093622 (PCT/US2013/074667), including a sequence encoding a Cas gene, a tracr (trans-activating CRISPR) sequence (e.g., tracrRNA or active portion tracrRNA), a tracr-mate sequence (including "direct repeat" and a partial direct repeat processed with a tracrRNA in the context of an endogenous CRISPR system), a guide sequence (also referred to as "spacer" in the context of an endogenous CRISPR system), or "RNA" (e.g., RNA that guides Cas such as Cas9, **e.g.,** CRISPR RNA and trans-activating (tracr) RNA or single guide RNA (sgRNA) (chimeric RNA)), or other sequences and transcripts from a CRISPR locus. In general, the CRISPR system is characterized by a sequence element (also referred to as protospacer in the context of an endogenous CRISPR system) that promotes formation of a CRISPR complex at a site of a target sequence. If a CRISPR protein is a Cpf1 protein, a tracrRNA is not required.

As used herein, the phrase "non-homologous end-joining (NHEJ)" or "non-homologous end-joining (NHEJ) pathway" refers to a pathway that repairs a double-strand break in DNA by directly joining ends of the break without requiring a homologous template. Template-independent rejoining of DNA ends by the NHEJ is a stochastic and error-prone repair process that introduces random microinsertion and microdeletion (indel) at a DNA break site. This method can be used to intentionally disrupt, delete, or change a reading frame of a targeted gene sequence. The NHEJ typically uses a short homologous DNA sequence, called microhomology, to induce a repair. The microhomology is often present in a single-strand overhang at an end of a double-strand break. When the overhang matches perfectly, the NHEJ usually repairs a cleavage accurately, while incorrect repair resulting in loss of a nucleotide can also occur, which is much more common when the overhang does not match.

As used herein, the term "DSB" means a double-strand break of a DNA.

As used herein, the term "indel" refers to a mutation that results in coexisting insertion and deletion and an increase or decrease in a nucleotide.

As used herein, the phrase "homology-directed repair" or "HDR" refers to a repair in such a way that a homologous sequence to a donor vector is recognized and a sequence of the vector is incorporated when a double-strand break of a DNA induced by a genome editing technology occurs. Insertion of a drug resistance gene in the donor vector allows drug selection of a cell in which the HDR has occurred. It is a repair mechanism utilizing a donor DNA and can also introduce a desired mutation in a target region.

As used herein, the phrase "microhomology-mediated end joining (MMEJ)" is a repair pathway that relies on a short complementary sequence (approximately 5 to 50 base pairs) between truncated ends at a DSB site, and is a technology of repairing a DSB using a sequence in which one strand is truncated and the other strand protrudes as a single strand.

As used herein, the phrase "homology arm sequence" means a polynucleotide suitable for targeting a gene to a genome through homologous recombination. Typically, two homology arms are flanked by a gene to be incorporated into a genome, and each homology arm contains sequences upstream or downstream of a locus at which integration occurs. Generally, the homology arm is about 50, about 60, about 70, about 80, about 90, about 100, about 200, about 300, about 400, about 500, about 600, about 700, about 800, about 900, about 1000, about 1500, about 2000 or more base pairs in length.

### (Preferred Embodiment)

Preferred Embodiments of the present disclosure will be described. Embodiments provided below are given for a better understanding of the present disclosure and the scope of the present disclosure should not be limited to the following description. Therefore, it is clear that those skilled in the art may make modifications as appropriate within the scope of the present disclosure, taking into account the description herein. The following embodiments of the present disclosure may also be used alone or in combination.

In one aspect of the present disclosure, a medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament including a Cas nuclease and/or a nucleic acid including a nucleic acid sequence encoding a Cas nuclease, and a nucleic acid including a nucleic acid sequence of a gRNA binding to the target nucleic acid sequence having the insertion or deletion, the Cas nuclease producing a cleavage site in the proximity of the target nucleic acid sequence having the insertion or deletion can be provided. In this case, the medicament can contain any component unless it impairs the effect of the medicament of the present disclosure.

In one embodiment, such a medicament can be used as a protein or nucleic acid medicament, and administration of the medicament of the present disclosure can produce a desired cleavage and repair in a cell, resulting in alteration, prevention, or treatment of a condition, disease, disorder, or symptom caused by a frameshift mutation.

In one embodiment, components constituting a medicament of the present disclosure can be combined into a mixture in which a plurality of active components are incorporated in a single formulation. In another embodiment, components constituting a medicament of the present disclosure may be present as separate compositions, each of which may be combined to form a medicament or provided as a kit including them. Thus, in another aspect of the present disclosure, a kit for altering, preventing, or treating a condition, disease, disorder, or symptom caused by a frameshift mutation due to insertion or deletion of one or more bases by cleaving at least one site of a target nucleic acid sequence in a nucleic acid within a cell, the kit including the above-mentioned pharmaceutical components of the present disclosure is provided.

In one embodiment, a medicament of the present disclosure may be in the form of a kit or a combination containing a protein in combination with an RNA. For example, in one embodiment, a medicament of the present disclosure may include a Cas nuclease, a gRNA binding to the target nucleic acid sequence having the insertion or deletion, and an instruction instructing that the Cas nuclease is administered so as to produce a cleavage site in the proximity of the target nucleic acid sequence having the insertion or deletion.

In one embodiment, a medicament of the present disclosure can be in the form of a kit or a combination containing a nucleic acid in combination with an RNA. For example, in one embodiment, a medicament of the present disclosure may include a nucleic acid including a nucleic acid sequence encoding a Cas nuclease, a gRNA binding to the target nucleic acid sequence having the insertion or deletion, and an instruction instructing that the Cas nuclease is administered so as to produce a cleavage site in the proximity of the target nucleic acid sequence having the insertion or deletion.

In one embodiment, a medicament of the present disclosure can also be provided as a nucleic acid medicament, for example, in one embodiment, a medicament of the present disclosure may include a nucleic acid including a nucleic acid sequence encoding a Cas nuclease, a nucleic acid including a nucleic acid sequence of a gRNA binding to the target nucleic acid sequence having the insertion or deletion, and an instruction instructing that the Cas nuclease is administered so as to produce a cleavage site in the proximity of the target nucleic acid sequence having the insertion or deletion.

In one embodiment, a medicament of the present disclosure can also be provided in the form including a protein and a vector. For example, in one embodiment, a medicament of the present disclosure may include a Cas nuclease, a nucleic acid including a nucleic acid sequence of a gRNA binding to the target nucleic acid sequence having the insertion or deletion, and an instruction instructing that the Cas nuclease is administered so as to produce a cleavage site in the proximity of the target nucleic acid sequence having the insertion or deletion.

In one embodiment, when the medicament as described above is provided as a kit, the Cas nuclease or the nucleic acid including a nucleic acid sequence encoding a Cas nuclease can be contained in a separate compartment from the gRNA binding to a target nucleic acid sequence having the insertion or deletion or the nucleic acid including a nucleic acid sequence of a gRNA binding to the target nucleic acid sequence having the insertion or deletion. In another embodiment, when such a medicament is provided as a combination, the Cas nuclease or the nucleic acid including a nucleic acid sequence encoding a Cas nuclease can be contained in combination with the gRNA binding to a target nucleic acid sequence having the insertion or deletion or the nucleic acid including a nucleic acid sequence of a gRNA binding to the target nucleic acid sequence having the insertion or deletion. In another embodiment, when such a medicament is provided as a mixture, the Cas nuclease or the nucleic acid including a nucleic acid sequence encoding a Cas nuclease can be contained in mixture with the gRNA binding to a target nucleic acid sequence having the insertion or deletion or the nucleic acid including a nucleic acid sequence of a gRNA binding to the target nucleic acid sequence having the insertion or deletion.

In one embodiment, when a medicament of the present disclosure is provided as a nucleic acid medicament or a vector, it can be provided using various delivery means. In this case, any method known in the art can be used, and a foreign DNA can be introduced into a cell by transfection or transduction. For example, various methods known in the art such as a calcium phosphate-DNA co-precipitation method, a DEAE-dextran-mediated transfection method, a polybrene-mediated transfection method, an electroshock method, a microinjection method, a liposome fusion method, or a lipofectamine and protoplast fusion method may be performed.

In another aspect of the present disclosure, a medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament including a vector, the vector including a construct that includes a promoter specific to the cell, a sequence encoding a Cas nuclease, a promoter enabling expression of a gRNA in the cell after introduction of the vector, and a sequence encoding a gRNA binding to the target nucleic acid sequence having the insertion or deletion, the construct being configured for the Cas nuclease to produce a cleavage site in the proximity of the target nucleic acid sequence having the insertion or deletion can be provided. In this case, the medicament can contain any component unless it impairs the effect of the medicament of the present disclosure.

In one embodiment of the present disclosure, a construct included in a medicament of the present disclosure can cleave a target nucleic acid sequence at a site in the proximity of an insertion or deletion site by a Cas nuclease. In one embodiment, a site to be cleaved by a Cas nuclease may be a site around a target nucleic acid sequence having insertion or deletion, as long as the site occurs on or upstream and/or downstream of the target nucleic acid sequence. In one embodiment, a cleavage site may be in the proximity of an insertion or deletion site, **e.g.,** within about 20 bases, about 15 bases, about 10 bases, about 9 bases, about 8 bases, about 7 bases, about 6 bases, about 5 bases, about 4 bases, about 3 bases, or about 2 bases from the insertion or deletion site. In one embodiment, when an insertion or deletion site is present in a conserved region, a cleavage site according to a technique of the present disclosure may be about 5 bases or more, about 10 bases or more, about 20 bases or more, about 30 bases or more, about 40 bases or more, about 50 bases or more, about 70 bases or more, or about 100 bases or more away from an insertion or deletion site.

In one embodiment, a site to be cleaved by a Cas nuclease can be a site flanked by an inserted or deleted base, **e.g.,** between an inserted base and a base adjacent upstream or downstream to the inserted base.

In one embodiment, a cleavage of a target nucleic acid sequence can be a double-strand break of a genomic nucleic acid in a cell, resulting in a blunt end.

In one embodiment, in a medicament of the present disclosure, a construct of the present disclosure is modified so that a reading frame shift responsible for a particular condition, disease, disorder, or symptom is restored as a result of working of a repair mechanism in a genome driven by cleavage by a Cas nuclease. EYS-S1653K, a highly prevalent mutation that is said to be present in approximately 12% of Japanese patients with retinitis pigmentosa, causes a frameshift mutation due to single base insertion in the 26th exon, resulting in interruption or termination of translation, which prevents normal expression of a protein and thus causes retinitis pigmentosa.

In one embodiment of the present disclosure, a target nucleic acid sequence in which a frameshift mutation has been caused by insertion or deletion of one or more bases can be restored to its normal sequence by producing a cleavage site in the proximity of the target nucleic acid sequence and subsequently repairing a genomic DNA. A cleavage site of the target nucleic acid sequence using genome editing may be anywhere as long as it is in the proximity of the target nucleic acid sequence having the insertion or deletion, but preferably the cleavage site can be made to be flanked by a site of the insertion or deletion. In one embodiment, a codon in which the mutation has occurred may be cleaved, or a sequence in the proximity of the codon in which the mutation has occurred may be cleaved.

In one embodiment of the present disclosure, a mutation to occur in a target nucleic acid sequence may preferably cause a frameshift mutation, and a base to be inserted or deleted may be only one base or, a plurality of bases such as two, four, five, seven, eight, or ten bases. In addition, in one embodiment, even if no frameshift mutation occurs such as insertion or deletion of three or six bases, a disease caused by translation of an aberrant protein due to insertion or deletion of one or more amino acids may also be encompassed by a disease of interest of the present disclosure.

Also, in one embodiment of the present disclosure, a base to be inserted or deleted may be contiguous or present at different positions.

In one embodiment, a gRNA can be designed so as to bind to a target nucleic acid sequence having insertion or deletion in order to produce a cleavage site in the proximity of the target nucleic acid sequence having insertion or deletion. Design of such a gRNA enables cleavage at a desired position.

As shown in Examples in the present disclosure, it is found that a target nucleic acid sequence will return to its normal sequence at a high probability by just producing a cleavage site in the proximity of the target nucleic acid sequence. Chemotherapy by administering a substance that induces exon skipping or a substance with a read-through activity has also been known as a treatment method for a hereditary disease caused by an out-of-frame mutation such as a frameshift mutation. Exon skipping is a technique whereby an exon containing an out-of-frame mutation is skipped, thereby converting the out-of-frame mutation to an in-frame mutation to obtain a protein encoded by mRNA lacking one or more of exons. The protein obtained by the exon skipping is shorter than a normal protein. The read-through activity is an activity that acts on a ribosome to allow it to read through a termination codon formed by an out-of-frame mutation and perform translation, so that a normal protein can be synthesized. In one embodiment of the present disclosure, a hereditary disease can be treated by just cleaving a target nucleic acid sequence without use of such a substance.

In one embodiment, when treating a disease or the like caused by a frameshift mutation by a technique according to the present disclosure, a cleavage site in a target nucleic acid sequence containing one or more insertions or deletions that is responsible for the disease may be repaired by non-homologous end joining (NHEJ), microhomology-mediated end joining (MMEJ), or a combination of NHEJ and MMEJ.

In one embodiment, when the cleavage site is repaired by MMEJ, a medicament, vector, or construct of the present disclosure can include homology arm sequences that are identical to sequences flanked by the cleavage site.

In one embodiment of the present disclosure, a medicament of the present disclosure includes a vector, the vector can include at least a construct including a promoter specific to the cell, a sequence encoding a Cas nuclease, a promoter enabling expression of a gRNA in the cell after introduction of the vector, and a sequence encoding a gRNA binding to a target nucleic acid sequence having the insertion or deletion. In one embodiment, the vector is not particularly limited, but can include, for example, an adeno-associated virus (AAV) vector.

In a genome editing gene therapy using an AAV, it is difficult to accurately replace a mutated sequence with a normal sequence by genome editing using a single AAV vector with a limited region that can be used for the gene therapy because of a large size of a Cas gene such as Cas9 required for the genome editing.

The present inventors have found that while conventional gene therapy is based on introduction of a full-length gene into a vector for treatment, a method in which genome editing is used to replace only an aberrant sequence of a gene (cut out the aberrant sequence and insert a normal sequence) can be employed to produce an AAV vector that is useful for gene therapy of a gene with a large size or for correcting a nucleic acid mutation (WO2020/096049). In one embodiment of the present disclosure, upon genome editing using such a single vector, a cleavage site can be produced in the proximity of a target nucleic acid sequence having insertion or deletion that induces a frameshift mutation to treat a hereditary disease of interest or the like.

Therefore, in another aspect of the present disclosure, a construct for altering, preventing, or treating a condition, disease, disorder, or symptom caused by a frameshift mutation due to insertion or deletion of one or more bases by cleaving at least one site of a target nucleic acid sequence in a nucleic acid within a cell, the construct being included in a vector, the construct including a promoter specific to the cell, a sequence encoding a Cas nuclease, a promoter enabling expression of a gRNA in the cell after introduction of the vector, and a sequence encoding the gRNA binding to the target nucleic acid sequence having the insertion or deletion, the construct being configured for the Cas nuclease to produce a cleavage site in the proximity of the target nucleic acid sequence having the insertion or deletion can be provided. A hereditary disease of interest can be treated by placing such a construct on a vector such as an AAV vector.

In another aspect of the present disclosure, a construct for altering, preventing, or treating a condition, disease, disorder, or symptom caused by a frameshift mutation due to insertion or deletion of one or more bases by cleaving at least one target site of a nucleic acid sequence in a nucleic acid within a cell, the construct including a promoter specific to the cell, a sequence encoding a Cas nuclease, a promoter enabling expression of a gRNA in the cell, and a sequence encoding the gRNA binding to the target nucleic acid sequence having the insertion or deletion, the construct being configured for the Cas nuclease to produce a cleavage site in the proximity of the target nucleic acid sequence having the insertion or deletion can also be provided. A construct according to the present disclosure does not necessarily need to be present in a vector. If the construct of the present disclosure can be delivered into a cell by a method other than introduction of a vector into the cell, a hereditary disease of interest can be treated.

Thus, according to still another aspect of the present disclosure, a method for altering, preventing, or treating a condition, disease, disorder, or symptom caused by a frameshift mutation due to insertion or deletion of one or more bases by cleaving at least one site of a target nucleic acid sequence in a nucleic acid within a cell, the method including introducing a vector into the cell, the vector including a construct that includes a promoter specific to the cell, a sequence encoding a Cas nuclease, a promoter enabling expression of a gRNA in the cell after introduction of the vector, and a sequence encoding the gRNA binding to the target nucleic acid sequence having the insertion or deletion; and placing the cell in a condition under which the cleavage occurs, the construct being configured for the Cas nuclease to produce a cleavage site in the proximity of the target nucleic acid sequence having the insertion or deletion is provided. Such a treatment method may be in human or in a non-human animal and may be an in vivo or in vitro method.

In the present disclosure, if a genome of a patient is examined in advance and it is determined that a disease, disorder, or symptom can be appropriately treated or prevented based on a target nucleic acid sequence that causes the disease, disorder, or symptom in a nucleic acid derived from the patient, a medicament or a method of the present disclosure can be used to treat or prevent the patient. Thus, according to a yet other aspect of the present disclosure, a method for altering, preventing, or treating a condition, disease, disorder, or symptom caused by a frameshift mutation due to insertion or deletion of one or more bases in a patient by cleaving at least one site of a target nucleic acid sequence in a nucleic acid within a cell, the method including sequencing the target nucleic acid sequence in a nucleic acid derived from the patient; selecting a gRNA that matches the target nucleic acid sequence in the patient based on the sequencing; and introducing a vector into the cell, the vector including a construct that includes a promoter specific to the cell, a sequence encoding a Cas nuclease, a promoter enabling expression of a gRNA in the cell after introduction of the vector, and a sequence encoding the gRNA binding to the target nucleic acid sequence having the insertion or deletion; and placing the cell in a condition under which the cleavage occurs, the construct being configured for the Cas nuclease to produce a cleavage site in the proximity of the target nucleic acid sequence having the insertion or deletion is provided. In one embodiment, the sequencing the target nucleic acid sequence can be performed using a method well known in the art. In one embodiment, the selecting a gRNA is based on sequencing results of the target nucleic acid sequence, and a degree of binding to the target nucleic acid sequence or editing efficiency based thereon can be evaluated to select a gRNA for a desired purpose. In this case, a score indicating the degree of binding to the target nucleic acid sequence or the editing efficiency based thereon as described below can also be used. In one embodiment, introducing a vector into the cell and the placing the cell in a condition under which the cleavage occurs can be performed using methods well-known in the art.

In one embodiment, according to a method of the present disclosure, efficacy can be evaluated by examining a patient with a genetic mutation that results in a frameshift in advance to determine whether or not a genome editing therapy is applicable to the patient. First, peripheral blood is collected from the patient to establish a lymphoblast, or a skin fragment is collected to establish a fibroblast, or a primary cultured cell or the like is prepared from blood, skin, or other biological specimens. By administering a genome editing vector to the thus-obtained cell and determining an effect of genome editing, a companion diagnostic can be made as to whether or not the patient can be treated. Even when a cell cannot be taken, if a genomic DNA or sequence information of the patient is known, it can be introduced into the cell and evaluated in the same manner. In another embodiment, a cleavage activity of a gRNA against a genomic DNA can also be evaluated in vitro.

In one embodiment, for a gRNA that binds to a target nucleic acid sequence, a gRNA design tool can be used to calculate a score such as a degree of binding to the target nucleic acid sequence or editing efficiency based thereon and employ a gRNA that has the score satisfying the predetermined value. For example, for a designable gRNA, efficacy thereof can be evaluated in silico by comparing the score using a design tool such as CRISPOR (http://crispor.tefor.net/), GT-scan2 (https://gt-scan.csiro.au/gt-scan2), CRISPick (GPP sgRNA Designer) (https://portals.broadinstitute.org/gppx/crispick/public). In one embodiment, a gRNA that exhibits a value of a score indicating editing efficiency of at least about 50% or more, preferably about 55% or more, about 60% or more, more preferably about 65% or more, about 70% or more, further preferably about 75% or more, about 80% or more, and even more preferably about 85% or more, or about 90% or more can be utilized. In another embodiment, a gRNA that exhibits a value of a score indicating specificity for a target nucleic acid sequence of at least about 90% or more, preferably about 95% or more, more preferably about 97% or more, further preferably about 98% or more, and even more preferably about 99% or more can be utilized. In one embodiment, for a gRNA binding to a target nucleic acid sequence, one that exhibits an MIT Specificity Score by CRISPOR of at least about 30% or more, preferably about 40% or more, further preferably about 50% or more, or a corresponding value thereto in another design tool can be utilized. When using CRISPOR, a gRNA binding to a target nucleic acid sequence can be selected based on specificity (off-target effect) and predictive efficiency (on-target cleavage) predicted by various algorithms. In one embodiment, an MIT Specificity Score can be used as an indication of specificity of a gRNA, and one having no off-target candidate or a score indicating cleavage efficiency can be further used if necessary. An MIT Specificity Score by CRISPOR can also be calculated based on the article such as Nature Biotechnology volume 31, pages 827-832 (2013).

In one embodiment, for a condition, disease, disorder, or symptom caused by a frameshift mutation due to insertion or deletion of one or more bases in a nucleic acid within a cell, if a gRNA exhibiting an MIT Specificity Score by CRISPOR of at least about 30% or more, preferably about 40% or more, further preferably about 50% or more, or a corresponding value thereto in another design tool can be designed as a gRNA binding to at least one site of a target nucleic acid sequence in the nucleic acid within the cell, the condition, disease, disorder, or symptom can be altered, prevented, or treated by using a method of the present disclosure, and the present disclosure can provide a medicament therefor.

In one embodiment of the present disclosure, examples of a subject that may be treated using a medicament of the present disclosure include human; non-human mammals such as cattle, pig, sheep, goat, rabbit, dog, cat, guinea pig, hamster, mouse, rat, and monkey; birds; fishes such as zebrafish; amphibians such as frog; reptiles; insects such as Drosophila; crustaceans, etc. Preferably, the subject is human and non-human mammals.

In one embodiment, a cell serving as a host cell may be a cell in vivo, an isolated primary cell, or a cultured cell. Since a genome is a common element in every cell, the genome editing technology developed by the present inventors can be applied to a cell in a nervous system tissue or any organ other than the nervous system tissue. For example, the cell may be, but is not limited to, a photoreceptor cell, a retinal pigment epithelial cell, a retinal bipolar cell, a retinal ganglion cell, a retinal horizontal cell, a retinal astrocyte, a retinal Muller cell, an amacrine cell, a retinal vascular endothelial cell, a corneal endothelial cell, a corneal epithelial cell, a corneal keratocyte, an iris epithelial cell, a ciliary epithelial cell, a fiber column cell, etc. The cell is preferably a retinal cell such as a photoreceptor cell since it is less likely to become cancerous. However, a cell in the brain and the spinal cord which are also central nervous system (a pyramidal cell, a stellate cell, a granule cell, a Purkinje cell, microglia, an oligodendrocyte, an astrocyte, etc.) can also be intended.

In one embodiment, the cell may be a cell of a normal subject, but is preferably a cell of a subject with a disease and more preferably a cell of a subject with a hereditary disease.

In one embodiment, a hereditary disease may be any disease caused by a genetic mutation. For example, a disease or a hereditary disease mainly encompasses, but is not limited to, an ocular disease such as a hereditary retinal degenerative disease, retinitis pigmentosa, macular degeneration, glaucoma, cataract, uveitis, optic neuritis, diabetic retinopathy, a retinal vascular occlusive disease, age-related macular degeneration, corneal dystrophy, bullous keratopathy, or corneal opacity; as well as hereditary deafness, Parkinson's disease, Huntington's disease, Alzheimer's disease, multiple sclerosis, rheumatoid arthritis, Crohn's disease, Peyronie's disease, ulcerative colitis, cerebral ischemia (stroke), myocardial infarction (heart attack), brain damage and/or spinal cord damage, reperfusion injury, ALS, Down's syndrome, cataract, schizophrenia, epilepsy, human leukemia and other cancers, and diabetes mellitus, and similar or any other hereditary diseases.

Retinitis pigmentosa (RP) is the second leading cause of acquired blindness in Japan and is a hereditary neurodegenerative disease for which there is no effective treatment. Although many of its etiologic mutations are still unknown, approximately 12% of Japanese patients with retinitis pigmentosa harbor an S1653K frameshift mutation in an EYS gene, which causes a frameshift mutation due to single base insertion in the 26th exon, resulting in interruption or termination of translation, which prevents normal expression of a protein and thus causes retinitis pigmentosa. In one embodiment of the present disclosure, a medicament of the present disclosure can be used to alter, prevent, or treat such a condition, disease, disorder, or symptom caused by a frameshift mutation due to insertion or deletion of one or more bases.

In one embodiment, a disease that can be treated by a medicament of the present disclosure is not particularly limited as long as it is a condition, disease, disorder, or symptom caused by a frameshift mutation due to insertion or deletion of one or more bases, and examples thereof include a hereditary disease such as those described above.

In one embodiment, a disease that can be treated by a technique of the present disclosure may include recessive hereditary hearing loss due to a mutation in a GJB2 gene, and the mutation in a GJB2 gene is known to be the most frequent causative gene of hereditary hearing loss. The GJB2 gene is a gene encoding a gap-binding protein (connexin 26), which is believed to play an important role in maintenance of a potassium ion in the inner ear. 35insG (p.Val13 frameshift mutation) and 239insA (p.Leu81 frameshift mutation) are known.

In another embodiment, a disease that can be treated by a technique of the present disclosure may include Rett syndrome due to a mutation in a FOXG1 gene, intellectual disability due to a mutation in a CHAMP1 gene, SELENON-related congenital myopathy due to a mutation in a SEPN1 gene, inclusion body myopathy due to a mutation in an HSPB8 gene, congenital disorder of deglycosylation due to a mutation in an NGLY1 gene, Zellweger syndrome due to a mutation in a PEX26 gene, Langer mesomelic dysplasia due to a mutation in a SHOX gene, rigidity and multifocal seizure syndrome due to a mutation in a BRAT1 gene, mucolipidosis II due to a mutation in a GNPTAB gene, congenital adrenal hyperplasia (CAH) due to a mutation in an NR0B1 gene, xeroderma pigmentosum due to a mutation in a POLH gene, glycogen storage disease type II due to a mutation in a GAA gene, Niemann-Pick disease type C1 due to a mutation in an NPC1 gene, and Usher syndrome caused due to a mutation in a USH2A gene. These hereditary diseases can be altered, prevented, or treated by cleaving a target nucleic acid sequence by a technology according to the present application.

In one embodiment, a condition, disease, disorder, or symptom caused by a frameshift mutation due to insertion or deletion of one or more bases can also be obtained using a known database. For example, if OMIM (https://omim.org/) is used as such a database, a disease to be examined (for example, "deafness, autosomal recessive", etc. for hearing loss which is an autosomal recessive disease) is searched in the latest update history. If a hearing loss disease is found in search results, a causative gene thereof is selected and only information related to a frameshift mutation is extracted from detailed information on the causative gene to thereby enable identification of a disease of interest and a frameshift mutation that is responsible for the disease.

In another aspect of the present disclosure, in order to produce a product for treating a disease that can be treated by a medicament of the present disclosure, a method for producing a product for altering, preventing, or treating a condition, disease, disorder, or symptom caused by a frameshift mutation due to insertion or deletion of one or more bases by cleaving at least one site of a target nucleic acid sequence in a nucleic acid within a cell, the method including: detecting a frameshift mutation due to insertion or deletion of one or more bases responsible for the condition, disease, disorder, or symptom; designing a construct based on the frameshift mutation, the construct including a promoter specific to the cell, a sequence encoding a Cas nuclease, a promoter enabling expression of a gRNA in the cell after introduction of the vector, and a sequence encoding a gRNA binding to the target nucleic acid sequence having the insertion or deletion; producing and inserting the construct into the vector; and producing a product including the vector, the construct being configured for the Cas nuclease to produce a cleavage site in the proximity of the target nucleic acid sequence having the insertion or deletion is provided. Such a method according to the present disclosure can be used as a research tool to produce a product for treating a disease to be treated by just finding a frameshift mutation due to insertion or deletion of one or more bases responsible for the disease and designing a construct appropriate for the mutation.

The frameshift mutation due to insertion or deletion of one or more bases responsible for the disease to be treated can be selected arbitrarily from the above-mentioned diseases or databases.

A construct and a vector that can be used in a production method of the present disclosure can be those described elsewhere herein, and a product produced by such a method can be used as a medicament to restore a mutated sequence in a cell to a normal sequence.

In one embodiment, a target nucleic acid sequence is a nucleic acid sequence to be cleaved using a medicament of the present disclosure, and can include insertion or deletion of one or more bases. In one embodiment, a target nucleic acid sequence can also have one or more (e.g., two, three, four, or five or more) base variants, preferably one or more (e.g., two, three, four, or five or more) base variants associated with a hereditary disease.

A mutation includes insertion or deletion, which can preferably result in a frameshift mutation. It is well known that a frameshift mutation due to insertion or deletion of one or more bases can cause disease, and a medicament, construct, and/or method of the present disclosure can be used to correct a DNA mutation in a cell in a subject by producing a cleavage site in the proximity of a target nucleic acid sequence having insertion or deletion of one or more bases in a genomic nucleic acid within a cell, and thus, can contribute to treatment of a disease. In another embodiment, a gene region or promoter region having no mutation can be a target of genome editing.

A length of a target nucleic acid sequence is not particularly limited, but can be 1 to about 1500 bases, preferably 1 to about 1200 bases, more preferably 1 to about 1000 bases, more preferably 1 to about 900 bases, more preferably 1 to about 800 bases, more preferably 1 to about 700 bases, more preferably 1 to about 600 bases, or more preferably 1 to about 500 bases in length.

In one embodiment, a sequence composed of about 17 to about 24 nucleotides in length at a 5' end of a PAM sequence upstream and/or downstream a target nucleic acid sequence within a cell (which may be the same sequence if present both upstream and downstream) can be used as a gRNA target sequence. In one embodiment, a medicament or construct of the present disclosure can have a sequence encoding a gRNA that recognizes the gRNA target sequence. In one embodiment, a gRNA target sequence in a vector of the present disclosure may be any sequence as long as it is recognized by a gRNA expressed by the vector.

In one embodiment, a gRNA target sequence and a PAM sequence can be adjacent to each other in a cell. A PAM (protospacer adjacent motif) sequence is a well-known sequence in the art that a nucleic acid within a cell has. Therefore, a PAM sequence in a nucleic acid within a cell varies with a type of Cas. For example, for saCas9 from Staphylococcus aureus, a PAM sequence includes 5'-NNGRRT-3' (R is A or G, N is any nucleotide) and is usually about 3 to 8 bases in length. Examples of the PAM sequence include, but are not limited to, NRG (R is A or G), NGA, NNAGAAW (W is A or T), NNNGMTT (M is A or C), or NNGRRT (R is A or G).

In one embodiment, a gRNA target sequence and a PAM sequence can include a sequence composed of about 17 to about 24 nucleotides in length that is complementary to a gRNA sequence and a PAM sequence composed of about 3 to about 8 nucleotides in length adjacent thereto.

In one embodiment, a medicament or construct of the present disclosure combines functions of expressing a Cas nuclease, expressing a gRNA, cleaving a nucleic acid region containing a target nucleic acid sequence in a genomic nucleic acid within a cell, and restoring a mutated sequence within the cell to a normal sequence in a single vector, and is capable of cleaving a desired nucleic acid precisely and conveniently.

In one embodiment, a medicament or construct of the present disclosure can also include inverted terminal repeats (ITRs) at both ends of a DNA strand of a vector, which are necessary for efficient proliferation of an AAV genome. In one embodiment, a vector of the present disclosure can include a Scaffold sequence.

In one embodiment of the present disclosure, a medicament or construct of the present disclosure has elements described herein preferably in order from a 5' end to a 3' end, but the order of arrangement of the elements is not limited as long as the effects of the present disclosure are achieved.

A nucleotide sequence in a vector can be mutated using a technique well-known in the art. A Cas nuclease has two DNA cleavage domains (HNH domain and RuvC domain) and can form a complex with a guide RNA (gRNA) to cleave a target portion of a nucleic acid.

In one embodiment, a Cas nuclease is not particularly limited, and, for example, Cas9 or Cas12 can be used. In one embodiment, Cas9 also includes any wild-type Cas9 nuclease and mutants thereof with a nuclease activity. Such mutants include nucleases in which 1, 2, 3, 4, 5, 6, 7, 8, 9, 10, 15, 20, 25, 30, 40, 45, or 50 amino acids of a wild-type Cas9 nuclease have been substituted, deleted, and/or added. Examples of a Cas9 nuclease include SpCas9 from Streptococcus pyrogenes, StCas9 from Streptococcus thermophilus, and SaCas9 from Staphylococcus aureus (Nature 520: 186-191, 2015). SaCas9 is preferred due to its small size and thus increased space available in an AAV vector.

Targeting of a nucleic acid is mediated by a sequence of at least about 17 to about 20 nucleotides at a 5' end of a gRNA. Such nucleotides are designed to be complementary to a complementary strand (opposite strand) to a nucleic acid sequence flanked by a PAM sequence in the nucleic acid (gRNA target sequence). Targeting occurs by interaction based on base pair complementarity between a complementary strand (opposite strand) to a nucleic acid sequence of a gRNA target sequence and the above-mentioned at least about 17 to about 20 nucleotides in a gRNA. Thus, a Cas nuclease produces a double-strand break specific for both a nucleic acid containing a target nucleic acid sequence in a cell and a vector.

A double-strand breaks are often located three bases upstream (5' end side) from a PAM sequence, but may be located at different sites within a gRNA target sequence. A gRNA recognizes a base sequence located on a 5' side of the PAM. Thus, a Cas nuclease specifically cleaves against a gRNA target sequence of a nucleic acid within a cell.

In one embodiment, a promoter specific to a cell (cell-specific promoter) can be selected as appropriate for a type of cell by those skilled in the art. The cell-specific promoter may be a naturally-occurring promoter or parts thereof, or may be a synthetic promoter.

Examples of such a cell-specific promoter include a naturally-occurring cell-specific promoter, a promoter having about 50 to about 150 contiguous base sequences in such a promoter, or a promoter composed of a base sequence that is about 90% or more identical to the about 50 to about 150 contiguous base sequences in such a promoter.

In one embodiment, for those used in the retina, examples of a naturally-occurring cell-specific promoter include, but are not limited to, a rhodopsin kinase promoter, an RPE65 promoter, a Best1 promoter, a mGluR6 promoter, a cone arrestin promoter, a CRALBP1 promoter, a Chx10 promoter, a rhodopsin promoter, a cone opsin promoter, and a recoverin promoter. Examples of a naturally-occurring cell-specific promoter for other organs include, but are not limited to, those selected from a synapsin I promoter, a myelin basic protein promoter, a neuron-specific enolase promoter, a calcium/calmodulin-dependent protein kinase II promoter, a tubulin alpha I promoter, a platelet-derived growth factor beta chain promoter, a glial fibrillary acidic protein (GFAP) promoter, an L7 promoter, and a glutamate receptor delta 2 promoter.

In one embodiment, a length of a cell-specific promoter is not particularly limited, but it is preferably short from the viewpoint of increasing space available in a vector. The length is preferably 800 bases or less, more preferably 700 bases or less, more preferably 600 bases or less, more preferably 500 bases or less, more preferably 400 bases or less, more preferably 300 bases or less, more preferably 200 bases or less, more preferably 150 bases or less, more preferably 120 bases or less, and further preferably 100 base or less.

When creating a synthetic promoter based on a sequence of a naturally-occurring promoter by substituting, deleting, or adding a portion of the sequence, for example, those skilled in the art can create a synthetic promoter with a shortened base length while maintaining a function of a cell-specific promoter by using a region that is highly conserved among species within the scope of ordinary skill.

An RNA polymerase III promoter is a promoter enabling expression of a gRNA in a mammalian cell after introduction of a vector. Examples of the RNA polymerase III promoter include a U6 promoter, an H1 promoter, and a 7SK promoter, but a U6 promoter is preferred from the viewpoint of driving a relatively short sequence. In one embodiment, a medicament or construct of the present disclosure may further include a modified sequence of a U6 promoter of a gRNA or a plurality of desired nucleic acids.

In one embodiment, a medicament or construct of the present disclosure can include a nuclear localization sequence (NLS) that results in nuclear translocation of a protein, and any known suitable NLS can be used. Many NLSs, for example, have a plurality of basic amino acids, referred to as bipartite basic repeats (Biochim. Biophys. Acta, 1991, 1071:83-101). An NLS containing bipartite basic repeats can be placed in any portion of a nucleic acid sequence, resulting in nuclear localization of an expressed protein.

In one embodiment, a medicament or construct of the present disclosure can further include any signal necessary for efficient polyadenylation of a transcript, transcription termination, a ribosome binding site, or translation termination. Such a site is well-known in the art, and those skilled in the art can select a suitable sequence.

Although a medicament or construct of the present disclosure is immediately applicable to many mutation-corrective therapies targeting a retinal photoreceptor cell, it is highly versatile enough to be applied to a treatment of a disease other than an ocular disease by using a small promoter that can be used in other tissues.

According to another aspect of the present disclosure, there is provided a kit for cleaving at least one site of a target nucleic acid sequence in a nucleic acid within a cell, the kit being provided with a medicament or construct of the present disclosure described above.

### (General technologies)

Molecular biological, biochemical, and microbiological procedures used herein are well known and common in the art, and are described in, for example, Sambrook J. et al. (1989). Molecular Cloning: A Laboratory Manual, Cold Spring Harbor and the 3rd Ed. (2001) thereof; Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990).PCR Protocols: A Guide to Methods and Applications, Academic Press; Ausubel, F.M. (1992) .Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, and Bessatsu Jikken Igaku "Idenshi donyu & hatsugen kaiseki jikken-hou", YODOSHA, CO., LTD., 1997, etc., each of which is incorporated herein by reference in its relevant parts (may be entirety).

For DNA synthesis techniques and nucleic acid chemistry to produce an artificially synthesized gene, gene synthesis and fragment synthesis services such as GeneArt, GenScript, or Integrated DNA Technologies (IDT) or those described in, for example, Gait, M., et al. J. (1985). Oligonucleotide Synthesis: A Practical Approach, IRL Press; Gait, M.J. (1990) . Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T.(I996). Bioconjugate Techniques, Academic Press, etc., each of which is incorporated herein by reference in its relevant parts, can be used.

As used herein, the term "or" is used when "at least one or more" of matters enumerated in a text can be employed. The same is true for "alternatively". When the phrase "within a range" of "two values" is specified herein, the range includes the two values themselves.

References cited herein such as the scientific literatures, patents, and patent applications are incorporated herein by reference in its entirety as if specifically set forth herein.

The present disclosure has been described with reference to preferred embodiments for ease of understanding. Hereinafter, the present disclosure will be described with reference to Examples, but the above description and Examples below are provided for illustrative purposes only and are not intended to limit the present disclosure. Accordingly, the scope of the present disclosure is not limited to embodiments or Examples specifically described herein, but is limited only by claims.

### [Example]

### (Example 1: Treatment of retinitis pigmentosa caused by EYS S1653K frameshift mutation)

### Construction of therapeutic vector

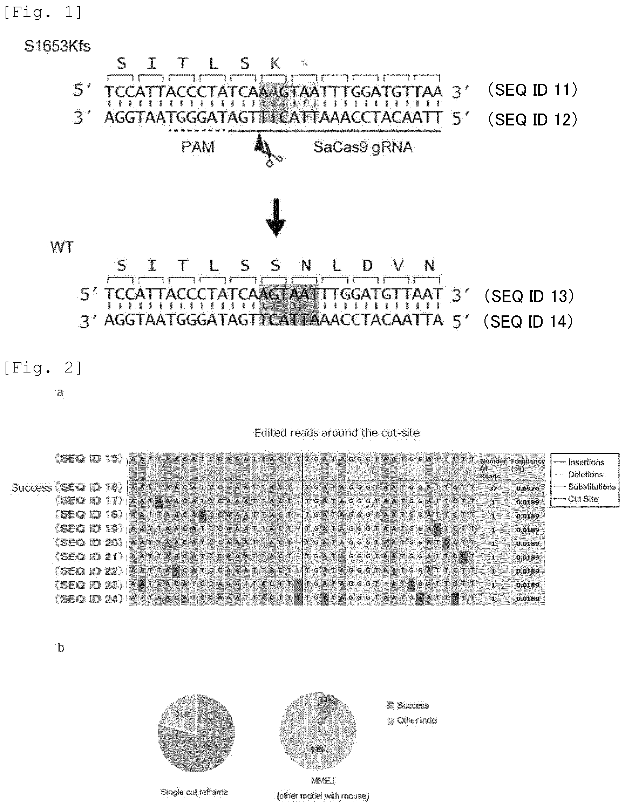

A gRNA oligo was subcloned into a pX601 vector (addgene #61591) to construct a genome editing vector for single-cut reframing. A sequence of a guide RNA was designed so that a site of an EYS S1653Kfs mutation was cleaved, as shown in Figure 1.

### On-target evaluation

To obtain a lymphoblastoid cell line (LCL), lymphocytes were transformed with an Epstein-Barr virus at the core facility of Tokyo Medical and Dental University. The LSL was cultured in an RPMI 1640 medium supplemented with 15% fetal bovine serum (FBS; Sigma-Aldrich) and 1% penicillin/streptomycin (Thermo Fisher Scientific, Waltham, MA) at 37°C and 5% CO₂. The LCL was introduced with a plasmid using an ECM630 electro cell manipulator (BTX, San Diego, CA) under conditions at 275 V, 100 Ω, and 1050 µF. A genomic DNA was extracted 48 hours after transfection using a QIAamp DNA mini kit (Qiagen, Hilden, Germany). The genomic DNA was analyzed on a MiSeq instrument and a MinION nanopore sequencer.

### Sequencing with MiSeq instrument

A forward primer (5'-TGAAGTCAAATCAGAATTCATCAAG-3') (SEQ ID NO. 1) and a reverse primer (5'-ATCAGTGGCATTCACAGAAGTG-3') (SEQ ID NO. 2) designed to contain a single cut (NHEJ) gene editing site were used and a sequence CGCTCTTCCGATCTCG (SEQ ID NO. 3) was added to a 5' end of the forward primer and a sequence CGCTTCCGATCTGAC (SEQ ID NO. 4) was added to a 5' end of the reverse primer to perform a bar code technology (HarismendyO., et al. (2011), Genome Biol., 12, R124., Forshew T., et al. (2012). Sci. Transl. Med., 4, 136ra168.). A first PCR reaction was performed in 10 µL containing 10 ng of a genomic DNA containing an edited and non-edited genomic DNA from the LCL, 5 µL of 2 x Platinum Multiplex PCR Master Mix (Thermo Fisher Scientific), and 0.1 pmol of each primer. A T100 Thermal Cycler (BIO-RAD, Hercules, CA) was used, cycling conditions were as follows: 30 cycles of 95°C for 2 minutes, 95°C for 30 seconds, 60°C for 90 seconds, and 72°C for 2 minutes, and then an elongation step was performed at 72°C for 10 minutes. A second PCR reaction was performed in 10 µL containing 1 µL of a product from the first PCR, 0.1 U KAPA HiFi HotStart DNA Polymerase (KAPA Biosystems, Wilmington, MA), 2 µL of 5 x KAPA HiFi Fidelity Buffer, 0.3 µL of 10 mM dNTPs, and 0.2 pmol of each primer. Cycling conditions were as follows: 4 cycles of 98°C for 45 seconds, 98°C for 15 seconds, 65°C for 30 seconds, and 72°C for 1 minute, and then an additional elongation step at 72°C for 1 minute was performed. The resulting library was purified using Agencourt AMPure XP (Beckman Coulter, Brea, CA) to remove a primer dimer. A MiSeq instrument (Illumina, San Diego, CA) was used to obtain 2 x 150-bp paired-end reads. CRISPECTOR was used to analyze a FASTQ sequence read file to detect and quantify an on-target genome editing activity using a paired treatment/control CRISPR experiment.

### Long read sequence analysis by MinION nanopore sequencer

Editing efficiencies were compared for two genome repair methods: a vector for single-cut reframing and an MMEJ vector. To reduce potential PCR bias, a PCR product of a gene editing region amplified by long PCR was sequenced using a MinION nanopore sequencer (Oxford Nanopore Technologies, Oxford, UK). The long PCR was performed using a forward primer (5'-AACTCATCAGCTGCACCG-3') (SEQ ID NO. 5) and a reverse primer (5'-GGGCTAGCAATCATGCACTTC-3') (SEQ ID NO. 6) designed to contain editing sites of the two methods. The thus-amplified PCR product of approximately 4.3 kbp was prepared using a Ligation Sequencing Kit (SQK-LSK110, Oxford Nanopore Technologies) according to a protocol of the kit. The resulting library was loaded into a Flongle Flow Cell (FLO-FLG001, Oxford Nanopore Technologies) and sequenced using MinKOWN. Total reads containing the above two primer sequences were extracted from the resulting FASTQ sequence read file, and reads containing successfully edited sequences were counted to determine their percentage accounted for of the total reads.

### RT-PCR

To produce a pcDNA 3.1-EYS WT+intron vector, a synthetic intron was inserted into a pcDNA 3.1(+)-EYS cDNA Vector (GenScript, Piscataway, NJ). A pcDNA 3.1-S1653Kfs+intron vector was obtained using a PrimeSTAR mutagenesis basal kit (Takara, Tokyo, Japan) with pcDNA 3.1-EYS WT+intron as a template. To produce a stable cell line, HEK293T cells (Thermo Fisher Scientific) were transfected with each plasmid using a PEI reagent (Polysciences Inc, Warrington, PA). The cells were divided into single cells and selected with 400 ug/ml G418 (Sigma-Aldrich). The cells were cultured in a DMEM medium (Thermo Fisher Scientific) supplemented with 10% fetal bovine serum (FBS; Sigma-Aldrich) and 1% penicillin/streptomycin (Thermo Fisher Scientific) at 37°C and 5% CO₂. A stable cell line was transfected with a single-cut frame or double-cut MMEJ gene editing plasmid using a PEI reagent (Polysciences Inc, Warrington, PA). Total RNA was extracted using a miRNeasy plus mini kit (Qiagen) according to an instruction of the kit 48 hours after the transfection. Then, 300 ng of a total RNA sample was reverse transcribed at 55°C for 30 min using SuperScript IV (Thermo Fisher Scientific) and random hexamer primers (Thermo Fisher Scientific). An RT-PCR reaction was performed using a KOD One DNA polymerase (Toyobo, Osaka, Japan) with EYS-specific primers Ex40-F (5'-GTTGGCCAGTGTCATGCTTC-3') (SEQ ID NO. 7) and Ex43-R (5'-CGCCAAGGTTGTAGCGAAGT-3') (SEQ ID NO. 8). GAPDH was used as an internal control using GAPDH-F (5'-TGACCACAGTCCATGCCATC-3') (SEQ ID NO. 9) and GAPDH-R (5'-TTACTCCTTGGGAGGCCATGTG-3') (SEQ ID NO. 10). The resulting PCR product was analyzed on an agarose gel.

### Result

In this Example, the new genome editing method was developed to repair a frameshift mutation by cleaving a genome at only one site (Figure 1). A therapeutic vector plasmid for an EYS S1653Kfs mutation was constructed and sequenced in vitro on an on-target site by a MiSeq Illumina platform. Sequence analysis showed 79% of successful reads (Figure 2). Comparisons with other genome editing were then made.

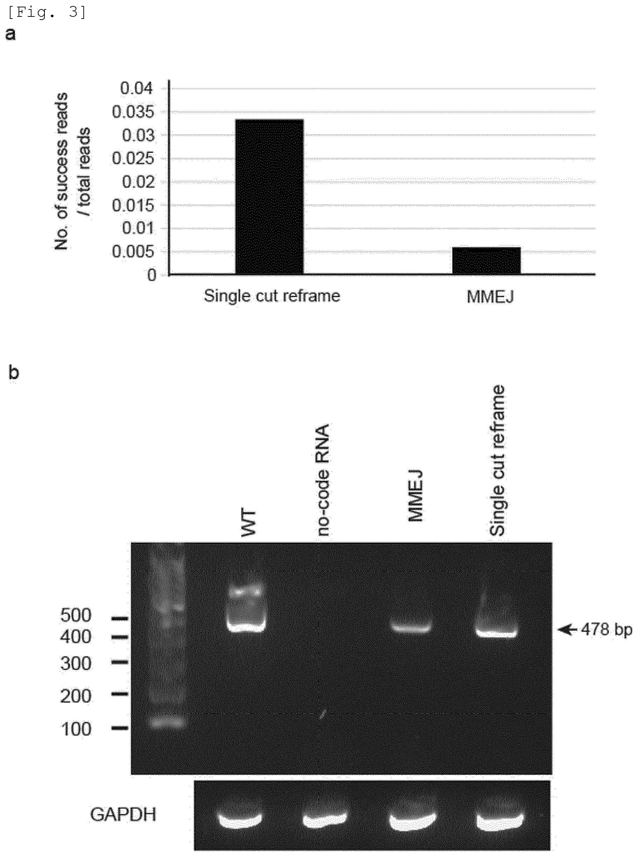

Long read sequencing analysis revealed that a success rate after a mutation repair with single-cut reframing was 3.34%, which was 5.58 times higher than that for MMEJ (0.599%) (Figure 3a). An mRNA analysis using RT-PCR also showed that the mutation repair with single-cut reframing is more efficient than the MMEJ (Fig. 3b). These results support that the method according to the present disclosure provides higher editing efficiency in a platform that repairs a frameshift mutation.

### (Example 2: Treatment of hereditary hearing loss caused by GJB2 gene mutation)

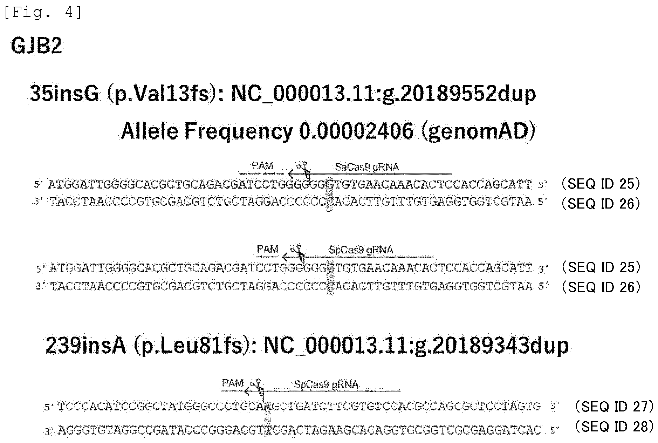

Hereditary hearing loss caused by a mutation on a GJB2 gene includes 35insG (p.Val13 frameshift mutation) and 239insA (p.Leu81 frameshift mutation), as shown in Figure 4.

A therapeutic vector containing a gRNA binding to a target nucleic acid sequence shown in Figure 4 is constructed in the same manner as in Example 1. Transformed cells are obtained in the same manner as in Example 1 and sequenced. Sequence analysis confirms that a mutation is repaired by single-cut reframing with high repair efficiency.

### (Example 3: Repair of frameshift by both NHEJ and MMEJ)

A gRNA oligo and a donor sequence were subcloned into a pX601 vector (addgene #61591) to construct a genome editing vector for single-cut reframing that performed a repair by both NHEJ and MMEJ pathways. A sequence of a guide RNA was designed so that a site of an EYS S1653Kfs mutation was cleaved in the same manner as in Example 1, as shown in Figure 1. A donor sequence having a normal sequence contains a microhomology arm and a gRNA target site.

On-target evaluation, sequencing with the MiSeq instrument, and sequencing analysis were performed in the same manner as in Example 1. The results are shown in Table 5. As shown in Figure 5, 73% of the reads were reframed by NHEJ and MMEJ.

### (Example 4: Repair of frameshift by MMEJ)

A genome editing vector for MMEJ reframing that repairs a frameshift by an MMEJ pathway is constructed in the same manner as in Example 3. A donor sequence having a normal sequence contains a microhomology arm and a gRNA target site. On-target evaluation, sequencing with the MiSeq instrument, and sequencing analysis were performed in the same manner as in Example 1 to verify whether or not the MMEJ reframes a frameshift mutation.

### (Example 5: Medicament with protein + gRNA)

A medicament containing a Cas nuclease and a gRNA is prepared. The Cas nuclease and the gRNA contained in such a medicament are designed to specifically bind to and cleave an insertion or deletion site that results in an etiologic frameshift mutation responsible for a disease to be treated. The medicament is administered to a subject to confirm a therapeutic effect.

### (Example 6: Medicament with nucleic acid + gRNA)

A medicament containing a gRNA and a nucleic acid including a nucleic acid sequence encoding a Cas nuclease is prepared. The gRNA and the nucleic acid including a nucleic acid sequence encoding a Cas nuclease contained in such a medicament are designed to specifically bind to and cleave an insertion or deletion site that results in an etiologic frameshift mutation responsible for a disease to be treated. The medicament is administered to a subject to confirm a therapeutic effect.

### (Example 7: Medicament containing vector)

A medicament containing a nucleic acid including a nucleic acid sequence encoding a Cas nuclease and a nucleic acid including a nucleic acid sequence of a gRNA is prepared. The nucleic acid including a nucleic acid sequence encoding a Cas nuclease and the nucleic acid including a nucleic acid sequence of a gRNA contained in such a medicament are designed to specifically bind to and cleave an insertion or deletion site that results in an etiologic frameshift mutation responsible for a disease to be treated. The medicament is administered to a subject to confirm a therapeutic effect.

### (Example 8: Treatment of Rett syndrome caused by FOXG1 gene mutation)

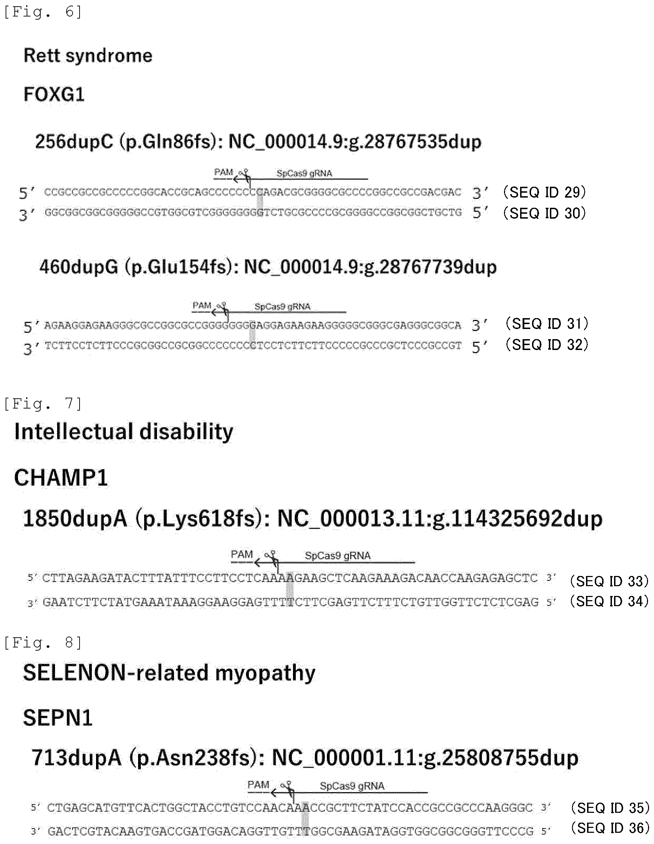

Rett syndrome caused by a mutation on a FOXG1 gene includes 256dupC (p.Gln86 frameshift mutation) and 460dupG (p.Gln154 frameshift mutation), as shown in Figure 6.

A therapeutic vector containing a gRNA binding to a target nucleic acid sequence shown in Figure 6 is constructed in the same manner as in Example 1. Transformed cells are obtained in the same manner as in Example 1 and sequenced. Sequence analysis confirms that a mutation is repaired by single-cut reframing with high repair efficiency.

### (Example 9: Treatment of intellectual disability caused by CHAMP1 gene mutation)

Intellectual disability caused by a mutation on a CHAMP1 gene includes 1850dupA (p.Lys618 frameshift mutation), as shown in Figure 7.

A therapeutic vector containing a gRNA binding to a target nucleic acid sequence shown in Figure 7 is constructed in the same manner as in Example 1. Transformed cells are obtained in the same manner as in Example 1 and sequenced. Sequence analysis confirms that a mutation is repaired by single-cut reframing with high repair efficiency.

### (Example 10: Treatment of SELENON-related congenital myopathy caused by SEPN1 gene mutation)

SELENON-related congenital myopathy caused by a mutation on a SEPN1 gene includes 713dupA (p.Asn238 frameshift mutation), as shown in Figure 8.

A therapeutic vector containing a gRNA binding to a target nucleic acid sequence shown in Figure 8 is constructed in the same manner as in Example 1. Transformed cells are obtained in the same manner as in Example 1 and sequenced. Sequence analysis confirms that a mutation is repaired by single-cut reframing with high repair efficiency.

### (Example 11: Treatment of inclusion body myopathy caused by HSPB8 gene mutation)

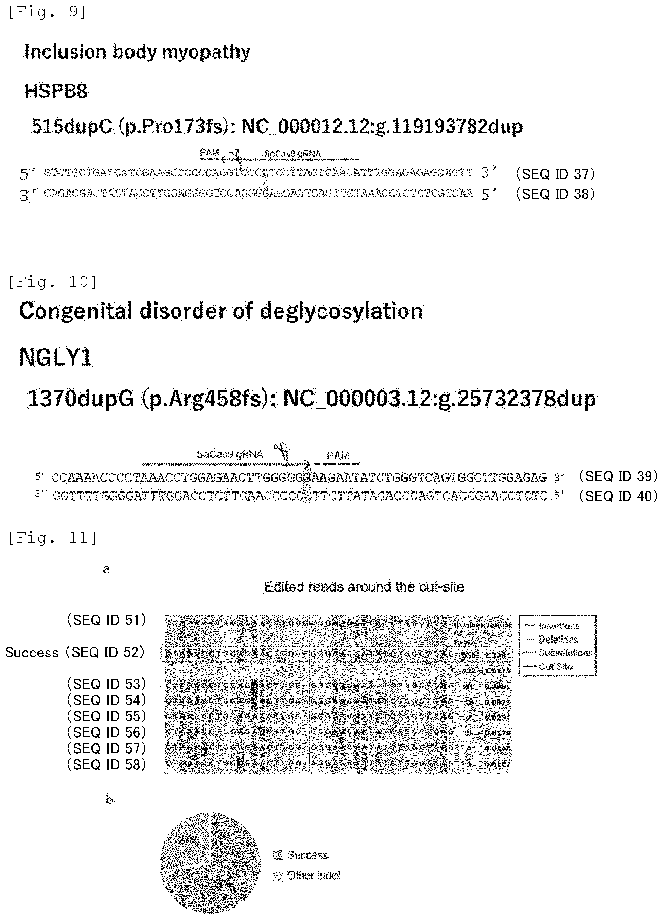

Inclusion body myopathy caused by a mutation on an HSPB8 gene includes 515dupC (p.Pro173 frameshift mutation), as shown in Figure 9.

A therapeutic vector containing a gRNA binding to a target nucleic acid sequence shown in Figure 9 is constructed in the same manner as in Example 1. Transformed cells are obtained in the same manner as in Example 1 and sequenced. Sequence analysis confirms that a mutation is repaired by single-cut reframing with high repair efficiency.

### (Example 12: Treatment of congenital disorder of deglycosylation due to NGLY1 gene mutation)

Congenital disorder of deglycosylation caused by a mutation on an NGLY1 gene includes 1370dupG (p.Arg458 frameshift mutation), as shown in Figure 10.

A therapeutic vector containing a gRNA binding to a target nucleic acid sequence shown in Figure 10 is constructed in the same manner as in Example 1. Transformed cells are obtained in the same manner as in Example 1 and sequenced. Sequence analysis confirms that a mutation is repaired by single-cut reframing with high repair efficiency.

### (Example 13: On-target evaluation using patient-derived skin fibroblast cell line)

### Construction of therapeutic vector

A gRNA oligo was subcloned into a pX601 vector (addgene #61591) to construct a genome editing vector for single-cut reframing. A sequence of a guide RNA was designed so that a mutation site was cleaved. Dermal fibroblasts from patients with congenital desugaring syndrome (NGLY1 mutation), Usher syndrome (USH1C mutation), and Rett syndrome (FOXG1 mutation) were obtained from the Coriell Institute (GM25391, GM24646, GM27411, respectively). The fibroblasts were cultured in a DMEM medium supplemented with 15% fetal bovine serum (FBS; Sigma-Aldrich) and 1% penicillin/streptomycin (Thermo Fisher Scientific) under an atmosphere at 37°C and 5% CO₂. A TransIT-2020 transfection reagent (Mirus Bio LLC, Madison, WI, USA) was used to transfect the fibroblasts with a plasmid. A genomic DNA was extracted 48 hours after the transfection using a QIAamp DNA mini kit (Qiagen). The genomic DNA was analyzed on the MiSeq instrument.

### Sequencing with MiSeq instrument

Forward primers (NGLY1, 5'-ACCTTGTAGACCCATTTCACCTC-3' (SEQ ID NO. 41); USH1C, 5'-CACACAGGAGAGGTCGTG-3' (SEQ ID NO. 42); FOXG1, 5'-CTCGTACTTGCCGTTCTTCTTC-3' (SEQ ID NO. 43); and Lpcat1, 5'-CTTCCTGCTCAAGGCCATCATG-3' (SEQ ID NO. 44)) and reverse primers (NGLY1, 5'-CAGAGGCAACTGTTTTTGTCAG-3' (SEQ ID NO. 45); USH1C, 5'-AGACCTGAAGCTGGGTCATCAATG-3' (SEQ ID NO. 46); FOXG1, 5'-CCCTGGACGGGGCTAAAGC-3' (SEQ ID NO. 47); and Lpcat1, 5'-CCCAGATTGGGATGTCTCTCTG-3' (SEQ ID NO. 48)) designed to contain single-cut (NHEJ) gene editing sites were used and a sequence CGCTTCCGATCTCTCTG (SEQ ID NO. 49) was added to a 5' end of each of the forward primers and a sequence CGCTTCCGATCTCTGAC (SEQ ID NO. 50) was added to a 5' end of each of the reverse primers to perform a bar code technology (Harrismendy O., et al. (2011), Genome Biol., 12, R124., Forshew T., et al. (2012). Sci. Transl. Med., 4, 136ra168.). A first PCR reaction was performed in 10 µL containing 10 ng of a genomic DNA containing an edited and non-edited genomic DNA from the LCL, 5 µL of 2 x Platinum Multiplex PCR Master Mix (Thermo Fisher Scientific), and 0.1 pmol of each primer. A T100 Thermal Cycler (BIO-RAD, Hercules, CA) was used and cycling conditions were as follows: 30 cycles of 95°C for 2 minutes, 95°C for 30 seconds, 60°C for 90 seconds, and 72°C for 2 minutes, and then an elongation step was performed at 72°C for 10 minutes. A second PCR reaction was performed in 10 µL containing 1 µL of a product of the first PCR, 0.1 U KAPA HiFi HotStart DNA Polymerase (KAPA Biosystems, Wilmington, MA), 2 µL of 5 x KAPA HiFi Fidelity Buffer, 0.3 µL of 10 mM dNTPs, and 0.2 pmol of each primer. Cycling conditions were as follows: 4 cycles of 98°C for 45 seconds, 98°C for 15 seconds,65°C for 30 seconds, and 72°C for 1 minute, and then an additional elongation step at 72°C for 1 minute was performed. The resulting library was purified using Agencourt AMPure XP (Beckman Coulter, Brea, CA) to remove a primer dimer. A MiSeq instrument (Illumina, San Diego, CA) was used to obtain 2 x 150-bp paired-end reads. CRISPECTOR was used to analyze a FASTQ sequence read file to detect and quantify an on-target genome editing activity using a paired treatment/control CRISPR experiment.

### On-target evaluation using HEK293T cell line

A gRNA oligo was subcloned into a pX601 vector (addgene #61591) to construct a genome editing vector for single-cut reframing. A sequence of a guide RNA was designed so that a site of a Lpcat1 rd11 mutation was cleaved. To construct a pCAG-neo Lpcat1^{rd11} vector, a mouse Lpcat1 cDNA (C130083H12; DNAFORM, Yokohama, Japan) was inserted into a pCAG-neo vector (Wako, Osaka, Japan). The pCAG-neo Lpcat1^{rd11} vector was obtained using a PrimeSTAR mutagenesis basal kit (Takara, Tokyo, Japan) with a pCAG-neo Lpcat1 vector as a template. A genome editing vector and a pCAG-neo Lpcat1^{rd11} plasmid were co-introduced into HEK293T cells (Thermo Fisher Scientific) using a PEI reagent (Polysciences Inc, Warrington, PA). The cells were cultured in a DMEM medium (Thermo Fisher Scientific) supplemented with 10% fetal bovine serum (FBS; Sigma-Aldrich) and 1% penicillin/streptomycin (Thermo Fisher Scientific) under an atmosphere at 37°C and 5% CO₂. The thus-transfected cells were collected 72 hours after the transfection. A genomic DNA was extracted using a QIAamp DNA mini kit (Qiagen). The genomic DNA was analyzed on the MiSeq instrument. A mRNA and a protein were extracted and analyzed by qRT-PCR and immunoblotting, respectively.

### Animal

For therapeutic genome editing, an AAV vector (1 x 10¹² gc mL⁻¹) was injected into the dorsal and ventral subretinal spaces of a Lpcat1^{rd11} mouse (Jackson Laboratory, Bar Harbor, ME) (1.5 µL per injection). Surgical procedures were performed after intraperitoneal administration of a mixture of ketamine (37.5 mg kg⁻¹) and medetomidine (0.63 mg kg⁻¹). The medetomidine was overridden by postoperative intraperitoneal administration of atipamezole (1.25 mg kg⁻¹). The mouse was handled in accordance with the ARVO Statement On the Use of Animals in Ophthalmic and Vision Research and The Guide for the Care and Use of Laboratory Animals at Tohoku University. All experimental procedures were approved by the relevant animal experiment committee of Nagoya University Graduate School of Medicine.

### Construction and purification of plasmid and AAV vector

To construct a therapeutic genome editing AAV vector for genome editing with single-cut reframing of a Lpcat1 rd11 mutation, a CMV promoter of pX601 with a Lpcat1 rd11 gRNA was replaced with a GRK1 promoter (GRK1-93). The thus-constructed plasmid vector was assembled into AAV2/8. Each vector and an AAV2rep/AAV8cap vector (pdp8; Plasmid Factory, Bielefeld Germany) were co-introduced into HEK293T cells (Thermo Fisher Scientific) using PEI (Polysciences Inc, Warrington, PA). AAV particles were extracted with PBS and purified using an AKTA prime plus chromatography system (Cytiva) on an AVB Separose HP column (Cytiva, Marlborough, MA).

### qRT-PCR

Total RNA was isolated using a miRNeasy plus mini kit (Qiagen) and reverse-transcribed using SuperScript IV (Thermo Fisher Scientific). qRT-PCR (QuantStudio 5; Thermo Fisher Scientific) was performed as follows: an initial denaturation step of 95°C for 20 seconds followed by 40 cycles of 95°C for 3 seconds and 60°C for 20 seconds. Gapdh (Mm99999915; Thermo Fisher Scientific) and Lpcat1 (Mm00461015; Thermo Fisher Scientific) Taqman probes were used.

### Western blot

A tissue was dissolved in a RIPA buffer and a total protein concentration was measured with a BCA protein assay kit (TaKaRa Bio, Kusatsu, Japan). A protein (10 µg each) was separated based on molecular weight by SDS-PAGE on a 5 to 20% Supersep Ace gel (Wako) and transferred to a PVDF membrane (Millipore, Billerica, MA). This was blocked with 5% skim milk for 1 hour, incubated with a rabbit anti-Lpcat1 antibody (HPA02268, 1/1000; Sigma-Aldrich) for 1 hour, and then incubated with a horseradish peroxidase (HRP)-labeled anti-rabbit IgG antibody (A0545, 1/2000; Sigma-Aldrich) for 1 hour. An immunogenic signal was detected with ECL prime (GE Healthcare). The membrane was removed, incubated with anti-β-actin (F5316, 1/2000; Sigma-Aldrich, St. Louis, MO), further incubated with an HRP-conjugated anti-mouse antibody (#31430, 1/2000; Thermo Fisher Scientific), and detected with ECL prime.

### Immunohistochemistry

An eyeball was fixed with 4% paraformaldehyde. The eyeball was then embedded in an OCT compound and sectioned using a cryostat to obtain a retinal section. For a reporter assay, the retinal section was stained only with DAPI (Vector Labs, Burlingame CA) for 45 minutes before imaging. For analysis of Lpcat1 expression, immunohistochemistry was performed using a TSA Plus Fluorescein System (KikoTech, Osaka, Japan) according to the manufacturer's instruction. The section was blocked with 1% skim milk for 1 hour, incubated with rabbit anti-Lpcat1 (HPA02268, 1/200; Sigma-Aldrich) for 1 hour, further incubated with an HRP-conjugated anti-rabbit IgG antibody (A0545, 1/2000; Sigma-Aldrich) for 1 hour, and then stained with a TSA reagent and/or DAPI for 45 minutes. An image was acquired with a Zeiss LSM780 confocal microscope (Carl Zeiss, Jena, Germany).

### Electrophysiological Evaluation

ERG and VEP were recorded using a PuREC (Mayo Corp., Inazawa, Japan) acquisition system and an LED stimulator (LS-100; Mayo). A mouse was dark-adapted overnight before an experiment, and the pupils were dilated with a combination of 0.5% tropicamide and 0.5% phenylephrine (Mydrin-P; Santen, Osaka, Japan). For standard single flash ERG, 10 levels of flash intensity from -7.0 to 2.0 log cd s m-2 in increments of 1.0 log were used. Surgical implantation of a VEP electrode was performed in the primary visual area 5 weeks after injection. One week later, data were recorded. For recording pVEP, vertical stripes with equal width of black (3 cd m-2) and white (159 cd m-2) (mean illuminance: 81 cd m-2) were used with different spatial resolutions (0.21, 0.14, 0.07, 0.05, 0.03, 0.02, 0.01 cycles per degree for Rhotvrm334/+ mouse). Amplitudes of a negative trough (P1-N1) and a positive peak (N1-P2) were plotted vertically as a function of logarithmic spatial resolution of stimuli.

### Behavioral experiment

An optokinetic response of a mouse to rotating sine wave gratings presented on monitors (mean luminance: 62 cd m-2) surrounding the mouse was observed (Optomotry, Cerebral Mechanics, Lethbridge, Canada). In this test, since a time-nasal motion implies a tracking response, it is possible to measure a visual acuity independently for the right and left eyes based on unequal sensitivity to a rotation direction of a pattern. A visual acuity datum is an average value of four runs for four consecutive days.

Three weeks after the AAV injection, a fear conditioning test was performed. In a training session, each mouse was placed in a stainless grid-floor shock chamber (21.5 cm wide x 20.5 cm deep x 30 cm high, O'HARA & CO., LTD.) installed in a sound reduction box, and a stimulus controller (FZ-LU, O'HARA & CO., LTD.) was used to control LED Light cue (535 nm, 0.015 cd m-2, 2.0 Hz, 5.0 sec) and 0.8 mA foot shocks (2.0 sec) were used for co-termination. These procedures were repeated five times at quasi-random intervals (70 to 140 seconds), after which the mouse was returned to a housing cage. During a test session, which was performed 24 hours after the training session, the mouse was surrounded by a sheet of white paper and returned to the same chamber as an environmentally altered chamber which was scented with a vanilla extract. After four minutes of environmental adaptation, light cues were given for 2 min. Freezing time was defined as no motion (less than 200 pixels and 1.0 second or more) and was recorded with a built-in infrared video camera. Freezing times of 2 minutes before and after presentation of the light cues were averaged using a preinstalled image processing software (O'HARA & CO., LTD.).

### Result

Therapeutic vector plasmids for mutations on NGLY1, USH1C, and FOXG1 genes were constructed and sequenced in vitro on an on-target site by a MiSeq Illumina platform (Figures 11 to 13). Sequence analysis detected 73% for NGLY1, 57% for USH1C, and 67% for FOXG1 of successful reads.

### (Example 14: Gene therapy of Lpcat1^{rd11} mutant mice)

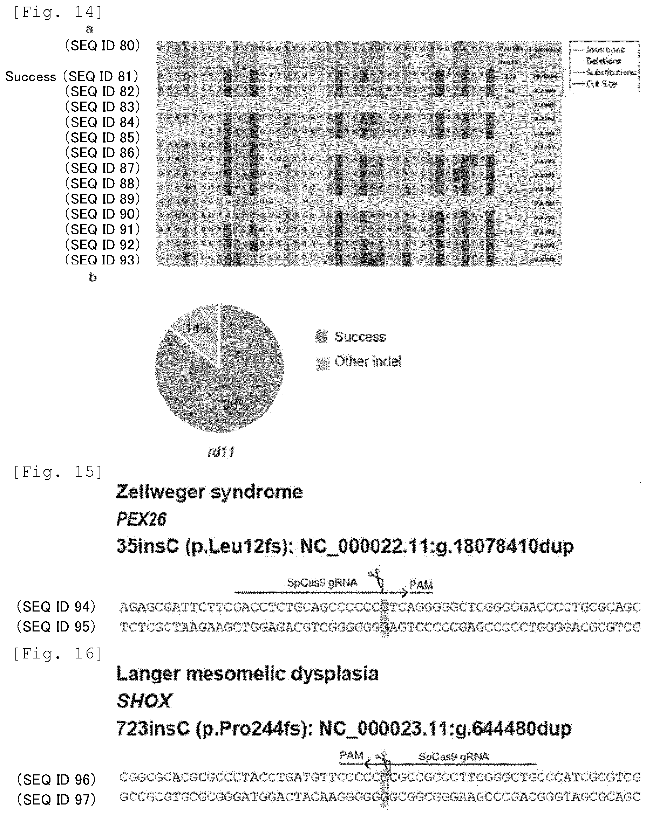

A Lpcat1^{rd11} mouse carries a frameshift mutation in a Lpcat1 gene associated with retinitis pigmentosa (RP). First, a therapeutic effect was confirmed by an in vitro experiment, which showed that the frameshift mutation was repaired in 86% of edited DNAs. Next, for an in vivo experiment using the Lpcat1^{rd11} mouse, a therapeutic AAV was produced and injected into the Lpcat1^{rd11} mouse to evaluate a therapeutic effect (Figure 14).

The repair of the frameshift mutation is also expected to be found in the in vivo experiment as is the case with the in vitro experiment. In addition, the therapeutic effect is expected to be found in qRT-PCR, immunoblotting, immunohistochemistry, behavioral, and electrophysiological tests.

### (Example 15: Treatment of Zellweger syndrome caused by PEX26 gene mutation)

Zellweger syndrome caused by a mutation on a PEX26 gene includes 35insC (p.Leu12 frameshift mutation), as shown in Figure 15.

A therapeutic vector containing a gRNA binding to a target nucleic acid sequence shown in Figure 15 is constructed in the same manner as in Example 1. Transformed cells are obtained in the same manner as in Example 1 and sequenced. Sequence analysis confirms that a mutation is repaired by single-cut reframing with high repair efficiency.

### (Example 16: Treatment of Langer mesomelic dysplasia caused by SHOX gene mutation)

Langer mesomelic dysplasia caused by a mutation on a SHOX gene includes 723insC (p.Pro244 frameshift mutation), as shown in Figure 16.

A therapeutic vector containing a gRNA binding to a target nucleic acid sequence shown in Figure 16 is constructed in the same manner as in Example 1. Transformed cells are obtained in the same manner as in Example 1 and sequenced. Sequence analysis confirms that a mutation is repaired by single-cut reframing with high repair efficiency.

### (Example 17: Treatment of rigidity and multifocal seizure syndrome caused by BRAT1 gene mutation)

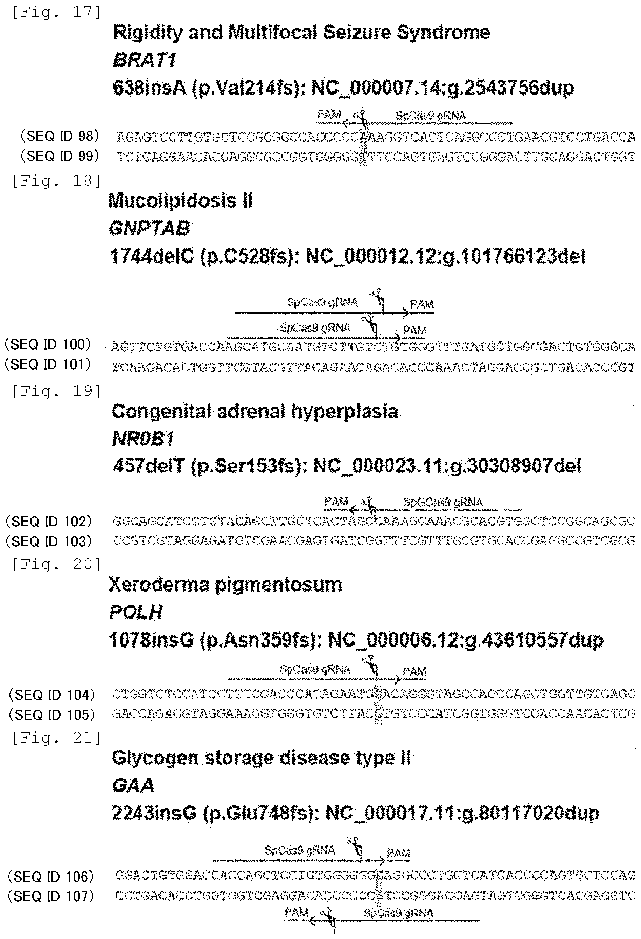

Rigidity and multifocal seizure syndrome caused by a mutation on a BRAT1 gene includes 638insA (p.Val214 frameshift mutation), as shown in Figure 17.

A therapeutic vector containing a gRNA binding to a target nucleic acid sequence shown in Figure 17 is constructed in the same manner as in Example 1. Transformed cells are obtained in the same manner as in Example 1 and sequenced. Sequence analysis confirms that a mutation is repaired by single-cut reframing with high repair efficiency.

### (Example 18: Treatment of mucolipidosis II caused by GNPTAB gene mutation)

Mucolipidosis II caused by a mutation on a GNPTAB gene includes 1744delC (p.C528 frameshift mutation), as shown in Figure 18.

A therapeutic vector containing a gRNA binding to a target nucleic acid sequence shown in Figure 18 is constructed in the same manner as in Example 1. Transformed cells are obtained in the same manner as in Example 1 and sequenced. Sequence analysis confirms that a mutation is repaired by single-cut reframing with high repair efficiency.

### (Example 19: Treatment of congenital adrenal hyperplasia (CAH) caused by NR0B1 gene mutation)

Congenital adrenal hyperplasia (CAH) caused by a mutation on an NR0B1 gene includes 457delT (p.Ser153 frameshift mutation), as shown in Figure 19.

A therapeutic vector containing a gRNA binding to a target nucleic acid sequence shown in Figure 19 is constructed in the same manner as in Example 1. Transformed cells are obtained in the same manner as in Example 1 and sequenced. Sequence analysis confirms that a mutation is repaired by single-cut reframing with high repair efficiency.

### (Example 20: Treatment of xeroderma pigmentosum caused by POLH gene mutation)

Xeroderma pigmentosum caused by a mutation on a POLH gene includes 1078insG (p.Asn359 frameshift mutation), as shown in Figure 20.

A therapeutic vector containing a gRNA binding to a target nucleic acid sequence shown in Figure 20 is constructed in the same manner as in Example 1. Transformed cells are obtained in the same manner as in Example 1 and sequenced. Sequence analysis confirms that a mutation is repaired by single-cut reframing with high repair efficiency.

### (Example 21: Treatment of glycogen storage disease type II caused by GAA gene mutation)

Glycogen storage disease type II caused by a mutation on a GAA gene includes 2243insG (p.Glu748 frameshift mutation), as shown in Figure 21.

A therapeutic vector containing a gRNA binding to a target nucleic acid sequence shown in Figure 21 is constructed in the same manner as in Example 1. Transformed cells are obtained in the same manner as in Example 1 and sequenced. Sequence analysis confirms that a mutation is repaired by single-cut reframing with high repair efficiency.

### (Example 22: Treatment of Niemann-Pick disease type C1 caused by NPC1 gene mutation)

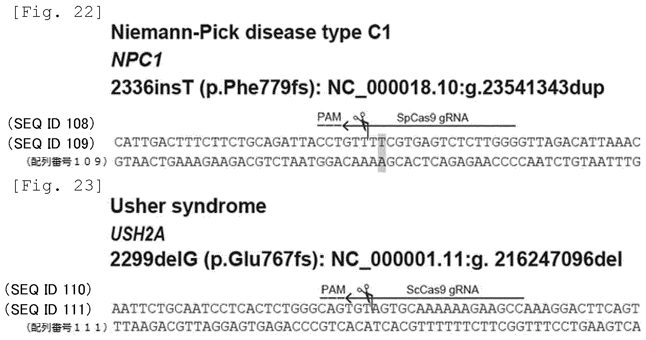

Niemann-Pick disease type C1 caused by a mutation on an NPC1 gene includes 2336insT (p.Phe779 frameshift mutation), as shown in Figure 22.

A therapeutic vector containing a gRNA binding to a target nucleic acid sequence shown in Figure 22 is constructed in the same manner as in Example 1. Transformed cells are obtained in the same manner as in Example 1 and sequenced. Sequence analysis confirms that a mutation is repaired by single-cut reframing with high repair efficiency.

### (Example 23: Treatment of Usher syndrome caused by USH2A gene mutation)

Usher syndrome caused by a mutation on a USH2A gene includes 2299delG (p.Glu767 frameshift mutation), as shown in Figure 23.

A therapeutic vector containing a gRNA binding to a target nucleic acid sequence shown in Figure 23 is constructed in the same manner as in Example 1. Transformed cells are obtained in the same manner as in Example 1 and sequenced. Sequence analysis confirms that a mutation is repaired by single-cut reframing with high repair efficiency.

### (Notes)

Although the present disclosure has been illustrated with reference to preferred embodiments of the present disclosure, it is understood that the scope of the present disclosure should be construed only by the claims. It is to be understood that the patents, patent applications, and other references cited herein should be incorporated herein by reference in its entirety as if the contents themselves are specifically set forth herein. This application claims priority to Japanese Patent Application No. 2022-30349 filed on February 28, 2022 with the Japan Patent Office, the contents of which are incorporated herein by reference in their entirety as if entire contents constitute contents herein.

### [Industrial Applicability]

The present disclosure is useful in the art of gene therapy since a gene that has undergone a frameshift mutation can be returned to a normal sequence or a sequence that is similar thereto by targeting a target nucleic acid sequence in a genome in a cell.

### [Sequence Listing Free Text]

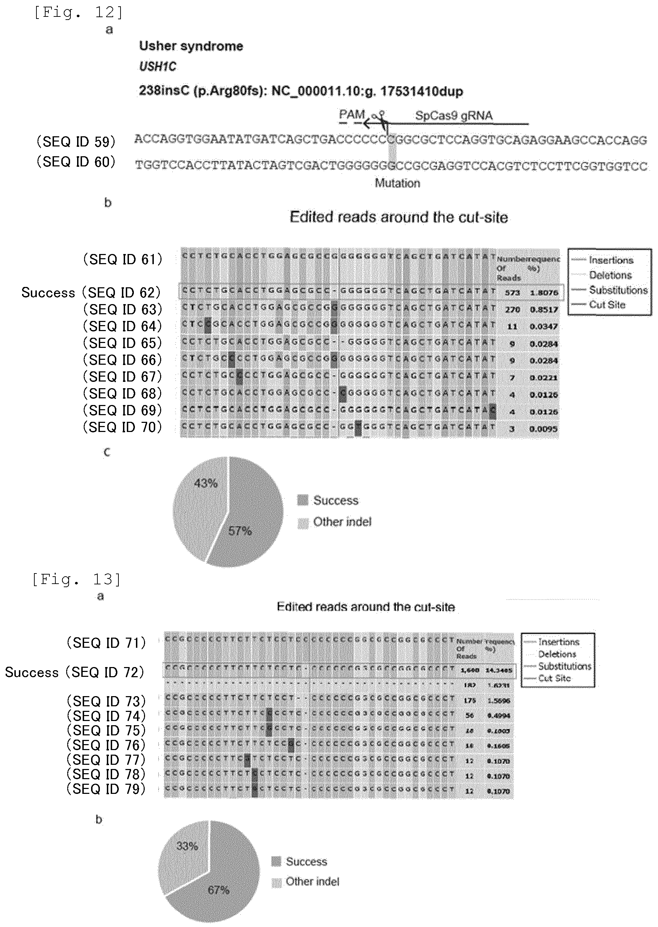

SEQ ID NO. 1: Forward primer used in Example 1
SEQ ID NO. 2: Reverse primer used in Example 1
SEQ ID NOs. 3 to 4: Barcode sequences used in Example 1
SEQ ID NO. 5: Forward primer used in Example 1
SEQ ID NO. 6: Reverse primer used in Example 1
SEQ ID NOs. 7 to 8: EYS-specific primers
SEQ ID NOs. 9 to 10: GAPDH-specific primers
SEQ ID NOs. 11 to 14: Sequences shown in Figure 1
SEQ ID NOs. 15 to 24: Sequences shown in Figure 2
SEQ ID NOs. 25 to 28: Sequences shown in Figure 4
SEQ ID NOs. 29 to 32: Sequences shown in Figure 6
SEQ ID NOs. 33 to 34: Sequences shown in Figure 7
SEQ ID NOs. 35 to 36: Sequences shown in Figure 8
SEQ ID NOs. 37 to 38: Sequences shown in Figure 9
SEQ ID NOs. 39 to 40: Sequences shown in Figure 10
SEQ ID NOs. 41 to 44: Forward primers used in Example 13
SEQ ID Nos. 45 to 48: Reverse primers used in Example 13
SEQ ID NOs. 49 to 50: Barcode sequences used in Example 13
SEQ ID NOs. 51 to 58: Sequences shown in Figure 11
SEQ ID NOs. 59 to 70: Sequences shown in Figure 12
SEQ ID NOs. 71 to 79: Sequences shown in Figure 13
SEQ ID NOs. 80 to 93: Sequences shown in Figure 14
SEQ ID NOs. 94 to 95: Sequences shown in Figure 15
SEQ ID NOs. 96 to 97: Sequences shown in Figure 16
SEQ ID NOs. 98 to 99: Sequences shown in Figure 17
SEQ ID NOs. 100 to 101: Sequences shown in Figure 18
SEQ ID NOs. 102 to 103: Sequences shown in Figure 19
SEQ ID NOs. 104 to 105: Sequences shown in Figure 20
SEQ ID NOs. 106 to 107: Sequences shown in Figure 21
SEQ ID NOs. 108 to 109: Sequences shown in Figure 22
SEQ ID NOs. 110 to 111: Sequences shown in Figure 23

## Claims

1. A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament comprising:
a Cas nuclease and/or a nucleic acid comprising a nucleic acid sequence encoding a Cas nuclease; and
a nucleic acid comprising a nucleic acid sequence of a gRNA binding to the target nucleic acid sequence having the insertion or deletion,
the Cas nuclease producing a cleavage site in a proximity of the target nucleic acid sequence having the insertion or deletion.

2. The medicament according to claim 1, wherein the medicament comprises a vector, the vector comprises a construct comprising a promoter specific to the cell, a sequence encoding the Cas nuclease, a promoter enabling expression of a gRNA in the cell after introduction of the vector, and a sequence encoding the gRNA binding to the target nucleic acid sequence having the insertion or deletion, and
the construct is configured for the Cas nuclease to produce a cleavage site in a proximity of the target nucleic acid sequence having the insertion or deletion.

3. The medicament according to claim 1 or 2, wherein the cleavage site is flanked by a site of the insertion or deletion.

4. The medicament according to any one of claims 1 to 3, wherein the condition, disease, disorder, or symptom caused by a frameshift mutation comprises retinitis pigmentosa caused by an EYS S1653K frameshift mutation.

5. The medicament according to any one of claims 1 to 4, wherein the vector comprises an adeno-associated virus (AAV) vector.

6. A construct for altering, preventing, or treating a condition, disease, disorder, or symptom caused by a frameshift mutation due to insertion or deletion of one or more bases by cleaving at least one site of a target nucleic acid sequence in a nucleic acid within a cell, the construct being comprised in a vector, the construct comprising a promoter specific to the cell, a sequence encoding a Cas nuclease, a promoter enabling expression of a gRNA in the cell after introduction of the vector, and a sequence encoding a gRNA binding to the target nucleic acid sequence having the insertion or deletion, and
the construct being configured for the Cas nuclease to produce a cleavage site in a proximity of the target nucleic acid sequence having the insertion or deletion.

7. The construct according to claim 6, wherein the cleavage site is flanked by a site of the insertion or deletion.

8. The construct according to claim 6 or 7, wherein the condition, disease, disorder, or symptom caused by a frameshift mutation comprises retinitis pigmentosa caused by an EYS S1653K frameshift mutation.

9. The construct according to any one of claims 6 to 8, wherein the vector comprises an adeno-associated virus (AAV) vector.

10. A construct for altering, preventing, or treating a condition, disease, disorder, or symptom caused by a frameshift mutation due to insertion or deletion of one or more bases by cleaving at least one site of a target nucleic acid sequence in a nucleic acid within a cell, the construct comprising a promoter specific to the cell, a sequence encoding a Cas nuclease, a promoter enabling expression of a gRNA in the cell, and a sequence encoding a gRNA binding to the target nucleic acid sequence having the insertion or deletion, and
the construct being configured for the Cas nuclease to produce a cleavage site in a proximity of the target nucleic acid sequence having the insertion or deletion.

11. The construct according to claim 10, wherein the cleavage site is flanked by a site of the insertion or deletion.

12. The construct according to claim 10 or 11, wherein the condition, disease, disorder, or symptom caused by a frameshift mutation comprises retinitis pigmentosa caused by an EYS S1653K frameshift mutation.

13. A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament comprising:
a Cas nuclease;
a gRNA binding to the target nucleic acid sequence having the insertion or deletion; and
an instruction instructing that the Cas nuclease is administered so as to produce a cleavage site in a proximity of the target nucleic acid sequence having the insertion or deletion.

14. The medicament according to claim 13, wherein the medicament is provided in a kit, the kit comprising:
the Cas nuclease; and
the gRNA binding to the target nucleic acid sequence having the insertion or deletion
in separate compartments.

15. The medicament according to claim 13 or 14, wherein the medicament is provided as a combination, the combination comprising:
the Cas nuclease; and
the gRNA binding to the target nucleic acid sequence having the insertion or deletion
in combination.

16. The medicament according to any one of claims 13 to 15, wherein the medicament is provided as a mixture, the mixture comprising:
the Cas nuclease; and
the gRNA binding to the target nucleic acid sequence having the insertion or deletion
mixed with each other.

17. A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament comprising:
a nucleic acid comprising a nucleic acid sequence encoding a Cas nuclease; and,
a gRNA binding to the target nucleic acid sequence having the insertion or deletion; and
an instruction instructing that the Cas nuclease is administered so as to produce a cleavage site in the proximity of the target nucleic acid sequence having the insertion or deletion.

18. The medicament according to claim 17, wherein the medicament is provided in a kit, the kit comprising:
the nucleic acid comprising a nucleic acid sequence encoding a Cas nuclease; and
the gRNA binding to the target nucleic acid sequence having the insertion or deletion
in separate compartments.

19. The medicament according to claim 17 or 18, wherein the medicament is provided as a combination, the combination comprising:
the nucleic acid comprising a nucleic acid sequence encoding a Cas nuclease; and
the gRNA binding to the target nucleic acid sequence having the insertion or deletion
in combination.

20. The medicament according to any one of claims 17 to 19, wherein the medicament is provided as a mixture, the mixture comprising:
the nucleic acid comprising a nucleic acid sequence encoding a Cas nuclease; and
the gRNA binding to the target nucleic acid sequence having the insertion or deletion
mixed with each other.

21. The medicament according to any one of claims 17 to 20, wherein the medicament comprises a vector, the vector comprises a construct comprising a promoter specific to the cell, a sequence encoding the Cas nuclease, a promoter enabling expression of a gRNA in the cell after introduction of the vector, and a sequence encoding the gRNA binding to the target nucleic acid sequence having the insertion or deletion, and
the construct is configured for the Cas nuclease to produce a cleavage site in a proximity of the target nucleic acid sequence having the insertion or deletion.

22. The medicament according to any one of claims 17 to 21, wherein the cleavage site is flanked by a site of the insertion or deletion.

23. The medicament according to any one of claims 18 to 22, wherein the condition, disease, disorder, or symptom caused by a frameshift mutation comprises retinitis pigmentosa caused by an EYS S1653K frameshift mutation.

24. A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament comprising:
a nucleic acid comprising a nucleic acid sequence encoding a Cas nuclease;
a nucleic acid comprising a nucleic acid sequence of a gRNA binding to the target nucleic acid sequence having the insertion or deletion; and
an instruction instructing that the Cas nuclease is administered so as to produce a cleavage site in a proximity of the target nucleic acid sequence having the insertion or deletion.

25. The medicament according to claim 24, wherein the medicament is provided in a kit, the kit comprising:
the nucleic acid comprising a nucleic acid sequence encoding a Cas nuclease; and
the nucleic acid comprising a nucleic acid sequence of a gRNA binding to the target nucleic acid sequence having the insertion or deletion
in separate compartments.

26. The medicament according to claim 24 or 25, wherein the medicament is provided as a combination, the combination comprising:
the nucleic acid comprising a nucleic acid sequence encoding a Cas nuclease; and
the nucleic acid comprising a nucleic acid sequence of a gRNA binding to the target nucleic acid sequence having the insertion or deletion
in combination.

27. The medicament according to any one of claims 24 to 26, wherein the medicament is provided as a mixture, the mixture comprising:
the nucleic acid comprising a nucleic acid sequence encoding a Cas nuclease; and
the nucleic acid comprising a nucleic acid sequence of a gRNA binding to the target nucleic acid sequence having the insertion or deletion
mixed with each other.

28. The medicament according to any one of claims 24 to 27, wherein the medicament comprises a vector, the vector comprises a construct comprising a promoter specific to the cell, a sequence encoding the Cas nuclease, a promoter enabling expression of a gRNA in the cell after introduction of the vector, and a sequence encoding the gRNA binding to the target nucleic acid sequence having the insertion or deletion, and
the construct is configured for the Cas nuclease to produce a cleavage site in a proximity of the target nucleic acid sequence having the insertion or deletion.

29. The medicament according to any one of claims 24 to 28, wherein the cleavage site is flanked by a site of the insertion or deletion.

30. The medicament according to any one of claims 24 to 29, wherein the condition, disease, disorder, or symptom caused by a frameshift mutation comprises retinitis pigmentosa caused by an EYS S1653K frameshift mutation.

31. A medicament for altering, preventing, or treating a condition, disease, disorder, or symptom caused by a frameshift mutation due to insertion or deletion of one or more bases by cleaving at least one site of a target nucleic acid sequence in a nucleic acid within a cell, the medicament comprising:
a Cas nuclease;
a nucleic acid comprising a nucleic acid sequence of a gRNA binding to the target nucleic acid sequence having the insertion or deletion; and
an instruction instructing that the Cas nuclease is administered so as to produce a cleavage site in a proximity of the target nucleic acid sequence having the insertion or deletion.

32. The medicament according to claim 31, wherein the medicament is provided in a kit, the kit comprising:
the Cas nuclease; and
the nucleic acid comprising a nucleic acid sequence of a gRNA binding to the target nucleic acid sequence having the insertion or deletion
in separate compartments.

33. The medicament according to claim 31 or 32, wherein the medicament is provided as a combination, the combination comprising:
the Cas nuclease; and
the nucleic acid comprising a nucleic acid sequence of a gRNA binding to the target nucleic acid sequence having the insertion or deletion
in combination.

34. The medicament according to any one of claims 31 to 33, wherein the medicament is provided as a mixture, the mixture comprising:
the Cas nuclease; and
the nucleic acid comprising a nucleic acid sequence of a gRNA binding to the target nucleic acid sequence having the insertion or deletion
mixed with each other.

35. The medicament according to any one of claims 31 to 34, wherein the medicament comprises a vector, the vector comprises a construct comprising a promoter specific to the cell, a sequence encoding the Cas nuclease, a promoter enabling expression of a gRNA in the cell after introduction of the vector, and a sequence encoding the gRNA binding to the target nucleic acid sequence having the insertion or deletion, and
the construct is configured for the Cas nuclease to produce a cleavage site in a proximity of the target nucleic acid sequence having the insertion or deletion.

36. The medicament according to any one of claims 31 to 35, wherein the cleavage site is flanked by a site of the insertion or deletion.

37. The medicament according to any one of claims 31 to 36, wherein the condition, disease, disorder, or symptom caused by a frameshift mutation comprises retinitis pigmentosa caused by an EYS S1653K frameshift mutation.

38. A method for producing a product for altering, preventing, or treating a condition, disease, disorder, or symptom by cleaving at least one site of a target nucleic acid sequence in a nucleic acid within a cell, the method comprising:
detecting a frameshift mutation due to insertion or deletion of one or more bases causing the condition, disease, disorder, or symptom;
designing a construct based on the frameshift mutation, the construct comprising a promoter specific to the cell, a sequence encoding a Cas nuclease, a promoter enabling expression of a gRNA in the cell after introduction of the vector, and a sequence encoding a gRNA binding to the target nucleic acid sequence having the insertion or deletion;
producing and inserting the construct into the vector; and
producing a product comprising the vector,
the construct being configured for the Cas nuclease to produce a cleavage site in a proximity of the target nucleic acid sequence having the insertion or deletion.

39. The method according to claim 38, wherein the cleavage site is flanked by a site of the insertion or deletion.

40. The method according to claim 38 or 39, wherein the vector comprises an adeno-associated virus (AAV) vector.
